# EUROPEAN PATENT SPECIFICATION

(11) **EP 4 440 407 B1**
(45) Date of publication and mention of the grant of the patent: **18.03.2026**
(21) Application number: 22847058.9
(22) Date of filing: 06.12.2022
(51) Int. Cl.: A61B 1/12, A61B 1/06, A61B 1/07, A61B 1/233, A61B 1/00, A61B 1/015

(54) **SYSTEMS, INSTRUMENTS AND METHODS FOR TREATING A SUBJECT**
SYSTEME, INSTRUMENTE UND VERFAHREN ZUR BEHANDLUNG EINER PERSON
SYSTÈMES, INSTRUMENTS ET PROCÉDÉS DE TRAITEMENT D'UN SUJET

(30) Priority: 23.12.2021 US 202117560346
(43) Date of publication of application: 09.10.2024
(73) Proprietor: excelENT, Inc., Durham, North Carolina 27709 (US)
(72) Inventor: MAZHAR, Kashif, Raleigh, North Carolina 27612 (US); THOMAS, Arlynn Celeste, Indian Land, South Carolina 29707 (US); BIETTE, Sean Christopher, Greenville, North Carolina 27858 (US); GARREN, Tiffany Mae, Marion, North Carolina 28752 (US); ZAROFF, Tyler, Concord, North Carolina 28025 (US); TANAKA, Martin Lyn, Cullowhee, North Carolina 27520 (US)
(74) Representative: Yeadon IP Limited
(86) International application number: PCT/US2022/080976
(87) International publication number: WO 2023/122425

(56) References cited:
- US-A1- 2014 243 640
- US-A1- 2015 282 701
- US-A1- 2019 328 412

## Description

### Background of the Invention

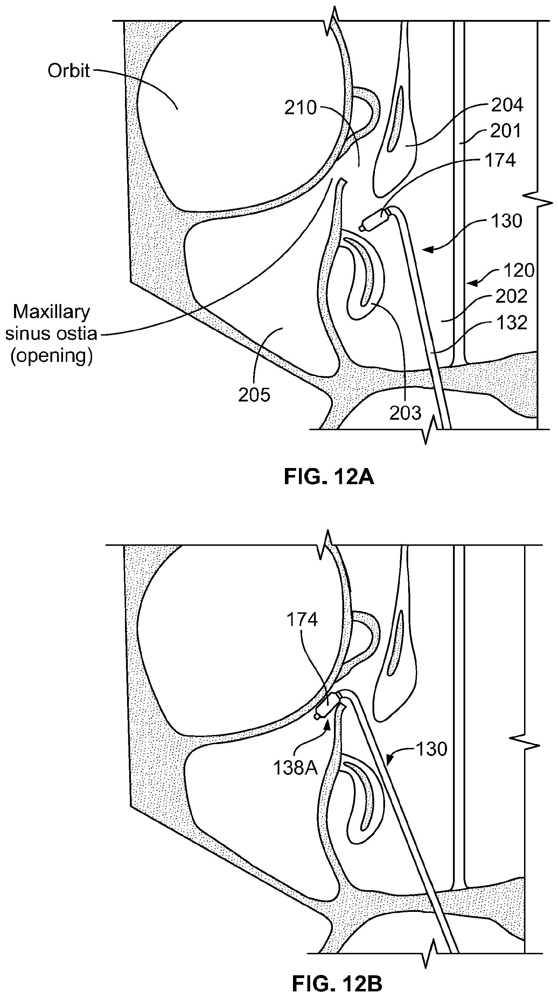

With reference to **FIG. 12A****,** the human nasal cavity is divided vertically by a wall of cartilage called the nasal septum **201.** On each side of the nasal septum is a nostril **202** through which the nasal cavity can be accessed. Opposite the septum on each lateral side of the nasal cavity are a series of turbinates (also called concha) comprising of inferior **203,** middle **204** and superior turbinate (not shown in any figures) as one goes backwards from the nostrils through the nasal cavity towards the throat. The turbinates are bony ridges that protrude into the nasal cavity.

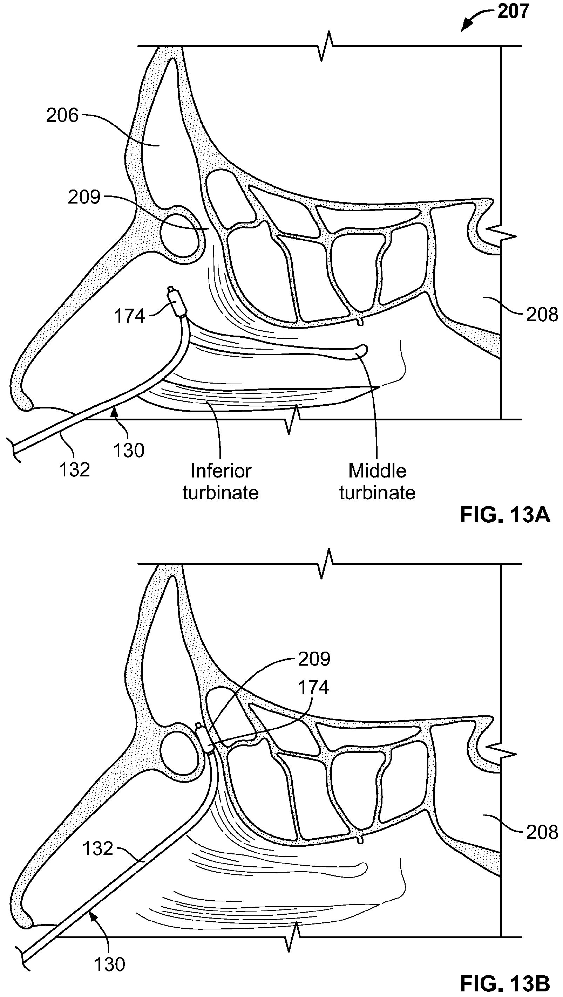

With reference to **FIG. 13A****,** the paranasal sinuses are a grouping of four pairs of air-filled cavities named after the facial bones in which they are located. The maxillary sinuses **205** are lateral to the nasal cavity in the region of the cheeks, the frontal sinuses **206** are in the forehead region above the eyes, the ethmoid sinuses **207** are located between the two eyes, and sphenoid sinuses **208** are located in the skull base under the pituitary gland. The ethmoid sinuses **207** are separated by a structure called the basal lamina into anterior and posterior ethmoid sinuses. The paranasal sinuses are lined by respiratory epithelium that secrete about a liter of sinus secretions everyday which drains out of the sinus cavity through small orifices called ostia and through the drainage pathways or outflow tract opening into the nasal cavity. The drainage pathway includes the ostia as well as a transition space in the region of the ostia called the "recess". A transition space in the proximity of the frontal sinus is called a frontal recess **209,** and a transition space in proximity of the sphenoid sinus and posterior ethmoid sinuses is called spheno-ethmoid recess (not shown in any figures), while the transition space in proximity to maxillary sinus is called the ethmoidal infundibulum **210.** The maxillary, anterior ethmoids and frontal sinuses drain into the nasal cavity from under the middle turbinate **204.** The posterior ethmoid and sphenoid sinuses drain through the outflow tract called spheno-ethmoid recess, which is located behind the superior turbinate. Efficient and effective transport of secreted mucus is essential for the health of the respiratory epithelial lining of the sinus cavity.

Inflammation of the mucosal linings of the sinus cavity is called sinusitis (or rhinosinusitis) and can be caused by multitude of causes such as anatomical abnormality, allergies, bacteria, or viruses that result in mild to severe symptomatic inflammation of the sino-nasal mucosa of one or more of the four paired sinus cavities (*i.e*., maxillary, ethmoid, frontal and sphenoid). This results in either the sinuses or their drainage pathways becoming either obstructed or compromised. Symptoms of sinusitis may include nasal obstruction, facial pressure/congestion/fullness, discolored nasal discharge and hyposmia.

Sinusitis is classified as acute sinusitis if less than four weeks in duration or as chronic sinusitis if lasting more than 12 weeks with or without acute exacerbation. Acute sinusitis is usually treated with medical management that includes oral antibiotics, oral antihistamine, topical or oral steroids. If non-responsive to medical management chronic sinusitis may need surgical intervention.

Balloon sinuplasty or balloon dilation of the sinus ostia and drainage pathways have been used to treat patients with chronic sinusitis. Balloon dilation generally involves an endoscopic, catheter-based inflatable balloon located at the distal end of the catheter to enlarge the affected sinus ostia or drainage pathways. Generally, the inflatable balloon is inserted into the constricted ostia or drainage pathways in a deflated state. Once correctly located and inflated, the balloon widens the walls of the sinus ostia or pathways without significant mucosal damage, resulting in lower operative morbidity and pain associated with the procedure for the patient.

Exemplary devices and methods particularly suited for the dilation of anatomic structures associated with maxillary and anterior ethmoid sinuses are disclosed, for example, in U.S. Patent No. 7,520,876 and U.S. Patent Application Publication No. 2008/0172033. Other systems have also been described for dilating the frontal sinus. For instance, U.S. Patent Application Publication No. 2008/0097295 discloses a frontal sinus guide catheter (FIG. 6B) and a method of treating the frontal sinuses (*e.g*., FIGS. 8B-8C). U.S. Patent Application Publication No. 2008/0125626 discloses another guide device (*e.g.,* FIGS 10C and 10C') for transnasal access to the frontal sinuses for treatment.

US20140243640 describes a balloon catheter system for measuring and locating catheters within blood vessels using electrical impedance measurements and light emission. The system includes an inflatable balloon with electrodes for measuring vessel dimensions, and an optical fiber (waveguide) that extends through the catheter structure. In use, when the balloon 65 is located in the vessel or lumen, the diameter, cross-sectional area and/or the volume of which is to be determined, the remote light source 71 is powered up. Light from the light source 71 is transmitted through the optical fibre cable 72, and in turn through the optical fibres 73 to emanate radially from the catheter member 3 through the radial bores 22 to identify the locations of the electrodes 7.

US 2019/328412 A1 discloses an instrument system for treating a subject with similar overall structure to the one described in this patent application including a base, probe, dilation balloon, and lighting system.

Existing balloon sinus dilation systems have several drawbacks. Some require multiple steps and multiple instruments. Some available sinus dilation systems require as many as eighteen steps to complete a sinus dilation procedure. Some sinus dilation systems need to be connected to an external light source for trans-illumination while others need to be connected to an image guidance system and hence need additional systems to be able to function.

### Summary of the Invention

The invention is defined by the independent claims. The dependent claims define advantageous embodiments.

According to some embodiments of the invention, an instrument system for treating a subject includes an instrument including a base configured to be gripped by an operator, and an elongate probe including a probe proximal end coupled with the base and extending to a probe distal end. The probe includes an aspiration lumen terminating at an inlet port proximate the probe distal end, and a delivery lumen terminating at an outlet port proximate the probe distal end. The instrument also includes a waveguide on the probe and having a light emitting end proximate the probe distal end.

In some embodiments, the instrument system is configured to: execute an aspiration operation wherein the instrument system draws material into the aspiration lumen through the inlet port; execute a fluid delivery operation wherein the instrument system flows a fluid through the delivery lumen and out through the outlet port; and execute an illumination operation wherein the instrument system transmits light through the waveguide and out through the light emitting end.

According to some embodiments, the instrument system is configured to enable the operator to execute the aspiration operation, the fluid delivery operation, and the illumination operation simultaneously.

In some embodiments, the instrument system is configured to enable the operator to execute the aspiration operation and the fluid delivery operation simultaneously.

According to some embodiments, the instrument includes a dilation balloon mounted on the probe proximate the probe distal end, and the instrument system is configured to execute a dilation operation wherein the dilation balloon is expanded.

In some embodiments, the instrument system is configured to enable the operator to execute the dilation operation simultaneously with at least one of the aspiration operation and the fluid delivery operation.

According to some embodiments, the instrument system is configured to enable the operator to execute the dilation operation, the aspiration operation, and the fluid delivery operation simultaneously.

In some embodiments, the instrument system is configured such that: the probe defines a probe longitudinal axis from the probe proximal end to the probe distal end; the base includes a handle configured to be gripped by a hand of the operator; and the handle has a handle axis extending at a transverse angle to the probe longitudinal axis.

According to further embodiments of the invention, an instrument system for treating a subject includes an instrument including a base configured to be gripped by an operator, and an elongate probe including a probe proximal end coupled with the base and extending to a probe distal end. The probe includes an aspiration lumen terminating at an inlet port proximate the probe distal end, and a delivery lumen terminating at an outlet port proximate the probe distal end. The instrument further includes a dilation balloon mounted on the probe proximate the probe distal end.

In some embodiments, the instrument system is configured to: execute an aspiration operation wherein the instrument system draws material into the aspiration lumen through the inlet port; execute a fluid delivery operation wherein the instrument system flows a fluid through the delivery lumen and out through the outlet port; and execute a dilation operation wherein the dilation balloon is expanded.

According to some embodiments, the instrument system is configured to enable the operator to execute the aspiration operation and the fluid delivery operation simultaneously.

In some embodiments, the instrument system is configured to enable the operator to execute the dilation operation simultaneously with at least one of the aspiration operation and the fluid delivery operation.

In some embodiments, the instrument system is configured to enable the operator to execute the dilation operation, the aspiration operation, and the fluid delivery operation simultaneously.

According to some embodiments, the instrument system includes: a rigid, elongate shaft, wherein the aspiration lumen is defined in shaft; and a delivery conduit extending through the aspiration lumen, wherein the delivery lumen is defined in delivery conduit. The dilation balloon is mounted on a distal end of the shaft.

The instrument system may include a supply of a medication fluidly connected to the delivery conduit.

According to method embodiments of the invention, a method for treating a subject includes providing an instrument including: a base configured to be gripped by an operator; and an elongate probe including a probe proximal end coupled with the base and extending to a probe distal end. The probe includes: an aspiration lumen terminating at an inlet port proximate the probe distal end; and a delivery lumen terminating at an outlet port proximate the probe distal end. The instrument also includes a waveguide on the probe and having a light emitting end proximate the probe distal end. The method further includes: executing an aspiration operation wherein the instrument system draws material into the aspiration lumen through the inlet port; executing a fluid delivery operation wherein the instrument system flows a fluid through the delivery lumen and out through the outlet port; and executing an illumination operation wherein the instrument system transmits light through the waveguide and out through the light emitting end.

In some embodiments, the method includes executing the aspiration operation, the fluid delivery operation, and the illumination operation simultaneously.

According to method embodiments of the invention, a method for treating a subject includes providing an instrument including: a base configured to be gripped by an operator; and an elongate probe including a probe proximal end coupled with the base and extending to a probe distal end. The probe includes: an aspiration lumen terminating at an inlet port proximate the probe distal end; and a delivery lumen terminating at an outlet port proximate the probe distal end. The instrument also includes a dilation balloon mounted on the probe proximate the probe distal end. The method further includes: executing an aspiration operation wherein the instrument system draws material into the aspiration lumen through the inlet port; executing a fluid delivery operation wherein the instrument system flows a fluid through the delivery lumen and out through the outlet port; and executing a dilation operation wherein the dilation balloon is expanded.

In some embodiments, the method includes executing the aspiration operation and the fluid delivery operation simultaneously.

According to some embodiments, the method includes executing the dilation operation simultaneously with at least one of the step of executing the aspiration operation and the step of executing the fluid delivery operation.

In some embodiments, the method includes executing the dilation operation, the aspiration operation, and the fluid delivery operation simultaneously.

According to some embodiments, an instrument system for treating a subject includes an instrument including a base, an elongate probe, a dilation balloon, and an integral lighting system. The base is configured to be gripped by an operator. The elongate probe includes a probe proximal end coupled with the base and extending to a probe distal end. The probe includes at least one lumen terminating at at least one port proximate the probe distal end. The dilation balloon is mounted on the probe proximate the probe distal end. The dilation balloon is expandable into an expanded configuration to execute a dilation operation. The integral lighting system includes a light source and a waveguide. The waveguide is on the probe and has a light emitting end proximate the probe distal end. The integral lighting system is configured to transmit light through the waveguide from the light source to the light emitting end.

In some embodiments, the integral lighting system includes a battery to power the light source.

In some embodiments, the light source and the battery are mounted in the base.

The light source may include a light emitting diode (LED).

The instrument system may further include an endoscope.

According to some embodiments, the instrument includes a heat sink member located adjacent the light source.

In some embodiments, the heat sink includes a through hole, and the waveguide extends from the light source through the through hole to the light emitting end.

In some embodiments, the heat sink is formed of metal.

According to some embodiments, a section of the waveguide extends through the dilation balloon, and when the light source is operated, light from the light source is transmitted through the waveguide, emitted radially outwardly through a sidewall of the waveguide, and transmitted through a side wall of the dilation balloon.

In some embodiments, the section of the waveguide emits an axially extending band of the light through the sidewall of the waveguide; and the band of light substantially spans a full axial length of the dilation balloon.

According to some embodiments, the probe includes an elongate, tubular outer shaft, wherein the outer shaft is rigid, and an elongate, tubular inner shaft extending through the outer shaft, wherein the inner shaft is malleable. The inner shaft includes a distal extension section extending distally beyond the outer shaft toward the probe distal end. The at least one lumen extends through the inner shaft. The dilation balloon is mounted on the distal extension section.

According to some embodiments, the at least one lumen includes a shared lumen terminating at a shared port proximate the probe distal end. The instrument system includes a suction source, an irrigation fluid source; and a fluid connector manifold fluidly connecting each of the suction source and the irrigation fluid source to the shared lumen. The suction source is operable to generate a negative pressure in the shared lumen to execute an aspiration operation wherein the instrument system draws material into the shared lumen through the shared port. The irrigation fluid source fluidly is operable to execute a fluid delivery operation wherein the instrument system flows a fluid through the shared lumen and out through the shared port.

In some embodiments, the instrument includes an integral aspiration controller operable to selectively control fluid flow between a suction source and the shared lumen, wherein the integral aspiration controller is mounted on the base, and the instrument system includes a delivery controller operable to selectively control fluid flow between the irrigation fluid source and the shared lumen.

In some embodiments, the instrument system includes a dilation controller operable to selectively inflate and deflate the dilation balloon, and the aspiration controller and the delivery controller are operable by the operator to execute the aspiration operation or the fluid delivery operation simultaneously while the dilation balloon is in its expanded configuration.

According to some embodiments, the at least one lumen and the at least one port include: an aspiration lumen terminating at an inlet port proximate the probe distal end; and a delivery lumen terminating at an outlet port proximate the probe distal end. The instrument system includes: a suction source fluidly connected to the aspiration lumen and operable to generate a negative pressure in the aspiration lumen to execute an aspiration operation wherein the instrument system draws material into the aspiration lumen through the inlet port; and an irrigation fluid source fluidly connected to the delivery lumen and operable to execute a fluid delivery operation wherein the instrument system flows a fluid through the delivery lumen and out through the outlet port.

According to some embodiments, the instrument includes an elastomeric atraumatic tip mounted on the probe distal end.

According to some embodiments, the instrument includes a visible dilation balloon position marker located on the probe at a position along a length of the dilation balloon.

According to some embodiments, a method for treating a subject includes providing an instrument including a base, an elongate probe, a dilation balloon, and an integral lighting system. The base is configured to be gripped by an operator. The elongate probe includes a probe proximal end coupled with the base and extending to a probe distal end. The probe includes at least one lumen terminating at at least one port proximate the probe distal end. The dilation balloon is mounted on the probe proximate the probe distal end. The dilation balloon is expandable into an expanded configuration to execute a dilation operation. The integral lighting system includes a light source and a waveguide. The waveguide is on the probe and has a light emitting end proximate the probe distal end. The integral lighting system is configured to transmit light through the waveguide from the light source to the light emitting end. The method further includes: executing an aspiration operation wherein the instrument system draws material into the at least one lumen through the at least one port; executing a fluid delivery operation wherein the instrument system flows a fluid through the at least one lumen and out through the at least one port; and executing an illumination operation wherein the instrument system transmits light through the waveguide and out through the light emitting end.

According to some embodiments, the method includes: trans-nasally inserting the probe into a sinus ostia or sinus drainage pathway of the subject such that the dilation balloon is disposed in the sinus ostia or sinus drainage pathway; and thereafter expanding the dilation balloon into an expanded configuration to dilate the sinus ostia or sinus drainage pathway.

In some embodiments, the method includes: trans-nasally inserting the probe into a eustachian tube of the subject such that the dilation balloon is disposed in the eustachian tube; thereafter expanding the dilation balloon into an expanded configuration to dilate the eustachian tube; and simultaneously with expanding the dilation balloon, executing the aspiration operation wherein the instrument system draws material into the at least one lumen through the at least one port to relieve excess pressure generated in the eustachian tube and/or a tympanic cavity in fluid communication with the eustachian tube by the expansion the dilation balloon.

According to some embodiments, a method for treating a includes: trans-nasally inserting a probe into a eustachian tube of the subject such that a dilation balloon on the probe is disposed in the eustachian tube; thereafter expanding the dilation balloon into an expanded configuration to dilate the eustachian tube; and while the dilation balloon is disposed in the eustachian tube and at least partially expanded, executing an aspiration operation wherein material is drawn out of the eustachian tube and/or a tympanic cavity in fluid communication with the eustachian tube to relieve excess pressure in the tympanic cavity generated by the expansion the dilation balloon.

In some embodiments, executing the aspiration operation includes drawing the material out of the eustachian tube and/or the tympanic cavity simultaneously with expanding the dilation balloon.

According to some embodiments, the probe has a probe distal end and includes a lumen terminating at a port proximate the probe distal end, the dilation balloon is mounted on the probe proximate the probe distal end, a suction source is fluidly connected to the lumen, and executing the aspiration operation includes using the suction source to generate a negative pressure in the lumen to draw material out of the eustachian tube and/or the tympanic cavity into the lumen through the port.

### Brief Description of the Drawings

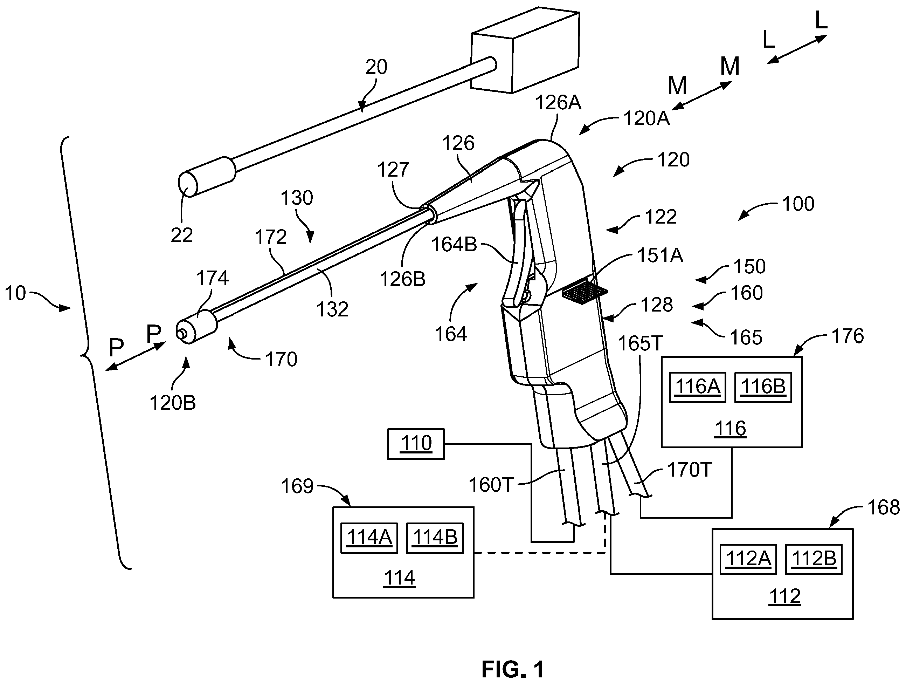

**FIG. 1** is front perspective, schematic view of a sinus treatment system according to some examples.

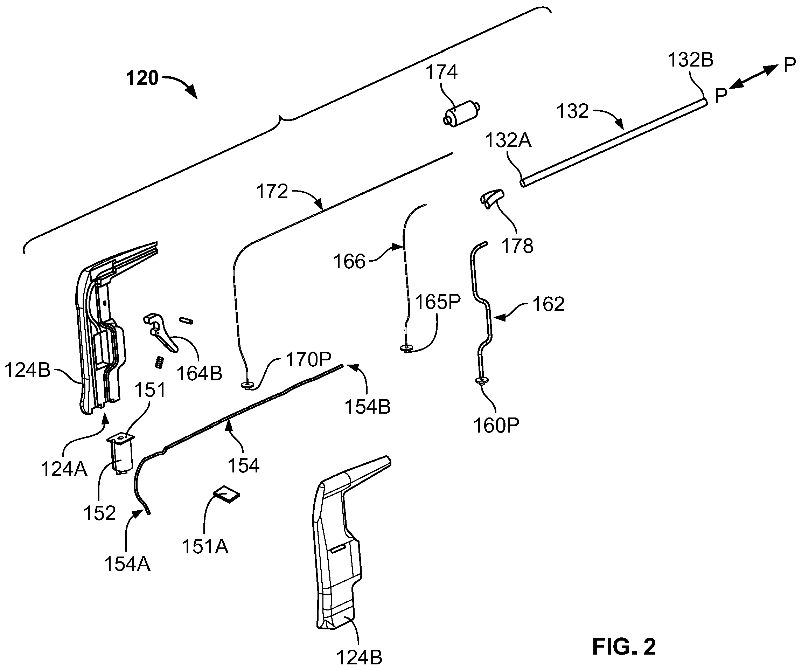

**FIG. 2** is an exploded, rear perspective view of an instrument forming a part of the sinus treatment system of **FIG. 1****.**

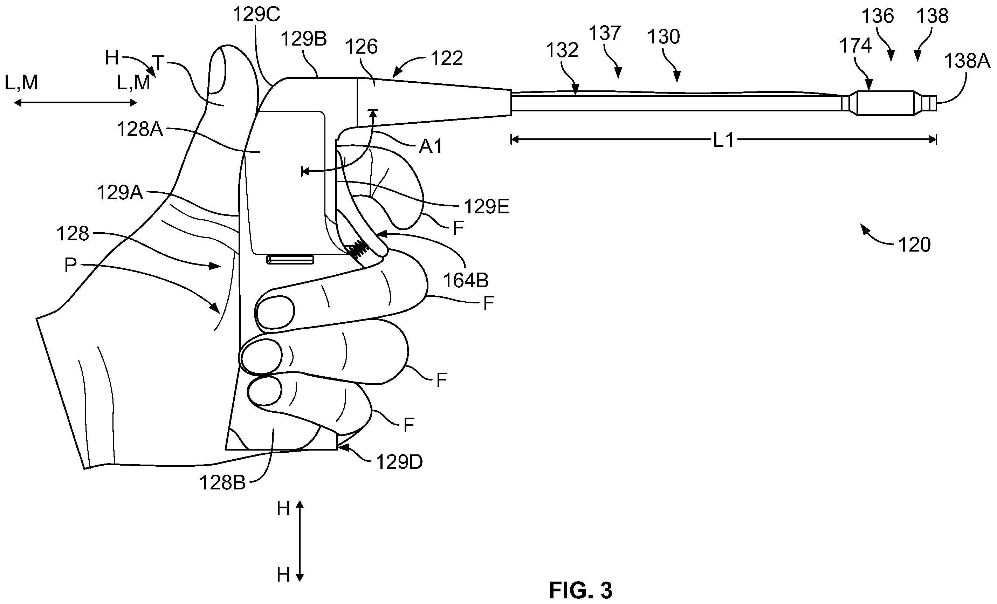

**FIG. 3** is a side view of the instrument of **FIG. 2****.**

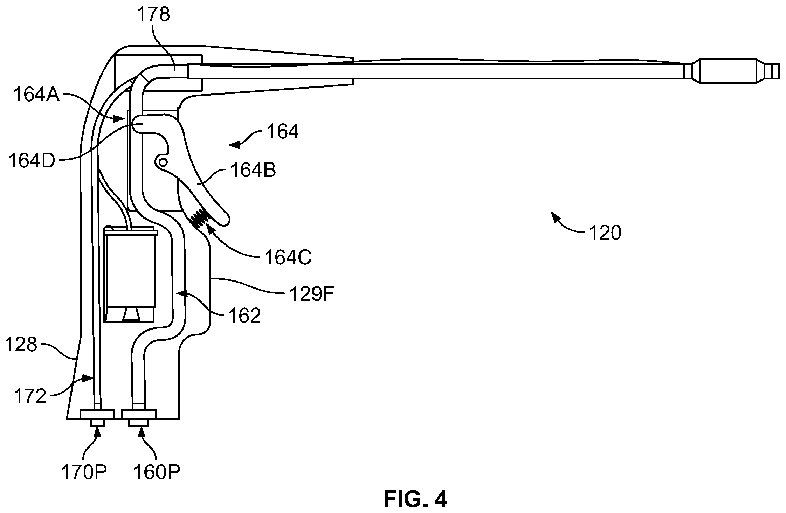

**FIG. 4** is a fragmentary, side view of the instrument of **FIG. 2****.**

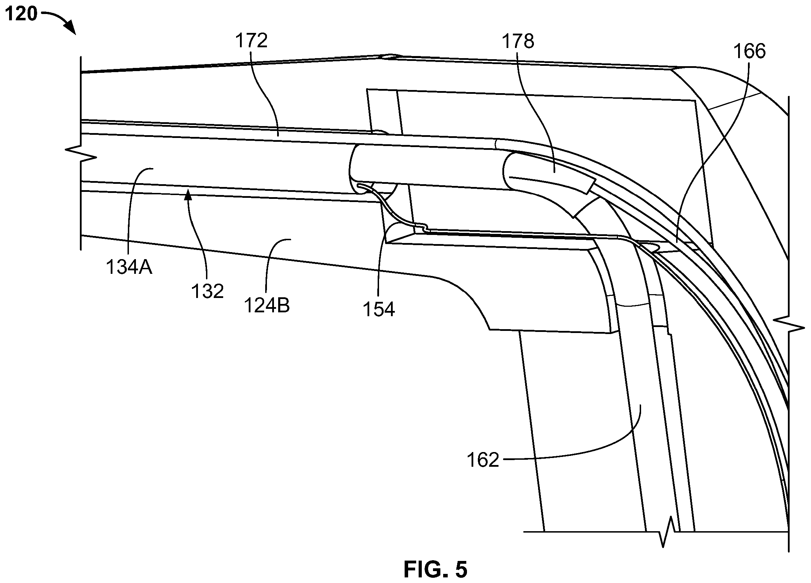

**FIG. 5** is a fragmentary, rear perspective view of the instrument **of** **FIG. 2****.**

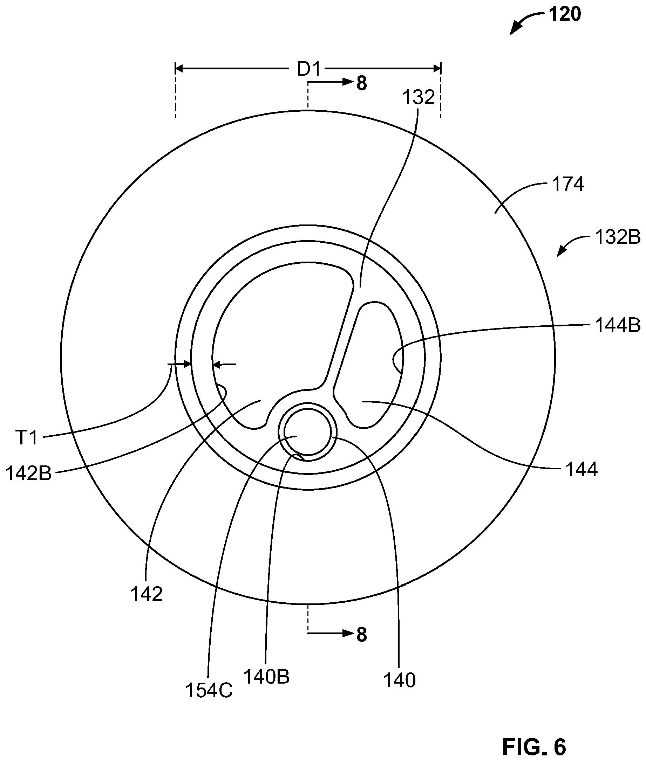

**FIG. 6** is a distal end view of the instrument of **FIG. 2****,** wherein a balloon of the instrument is in an expanded position.

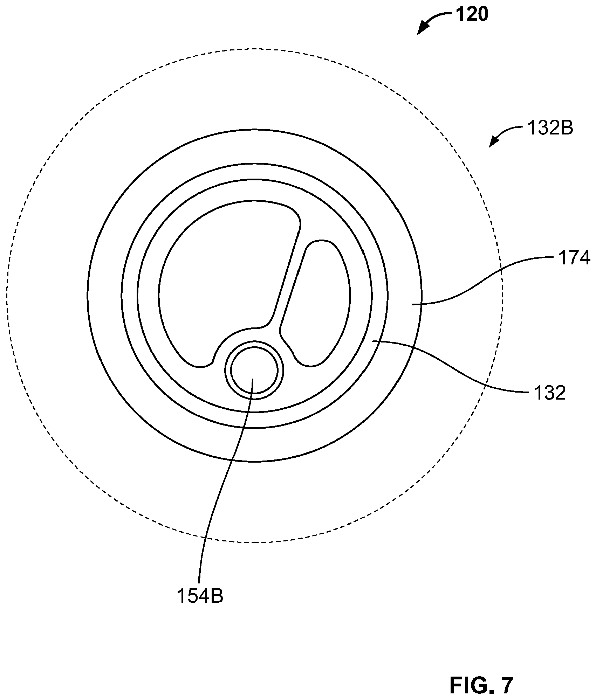

**FIG. 7** is a distal end view of the instrument of **FIG. 2****,** wherein the balloon is in a deflated position.

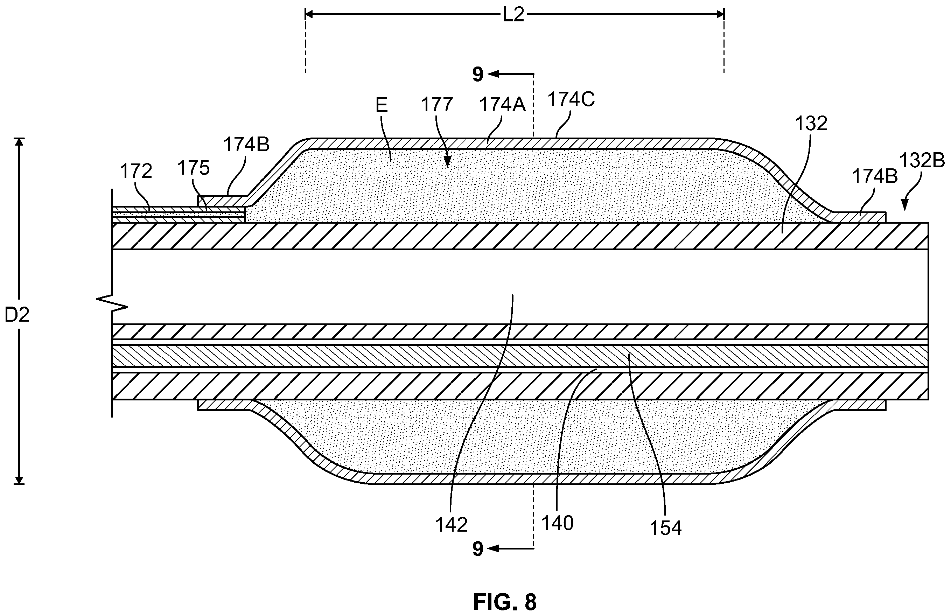

**FIG. 8** is a cross-sectional view of the instrument of **FIG. 2** taken along the line **8-8** of **FIG. 6****,** wherein the balloon is in the expanded position.

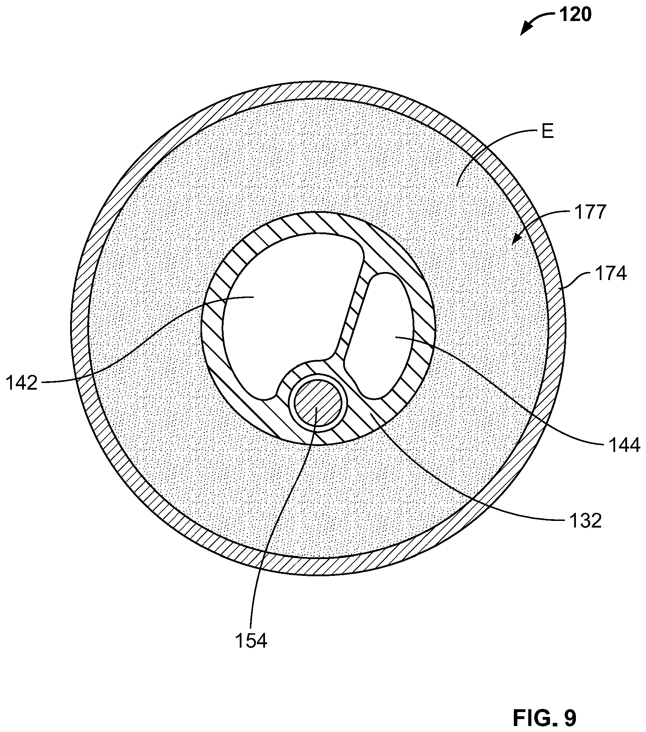

**FIG. 9** is a cross-sectional view of the instrument of **FIG. 2** taken along the line **9-9** of **FIG. 8****,** wherein the balloon is in the expanded position.

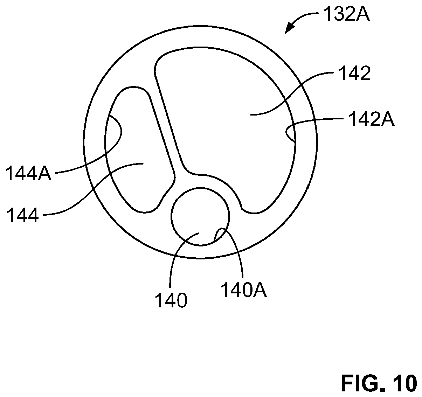

**FIG. 10** is a proximal end view of a shaft forming a part of the instrument of **FIG. 2****.**

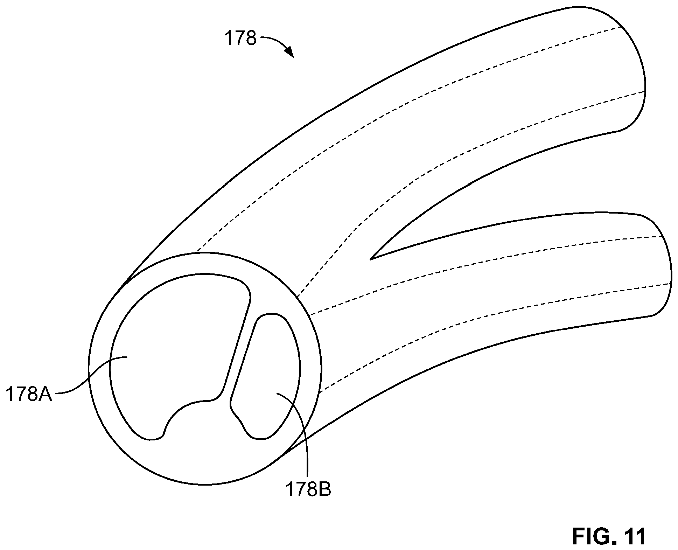

**FIG. 11** is a rear perspective view of a fitting forming a part of the instrument of **FIG. 2****.**
**FIGS. 12A-13F** illustrate methods using the sinus treatment system of **FIG. 1****.**

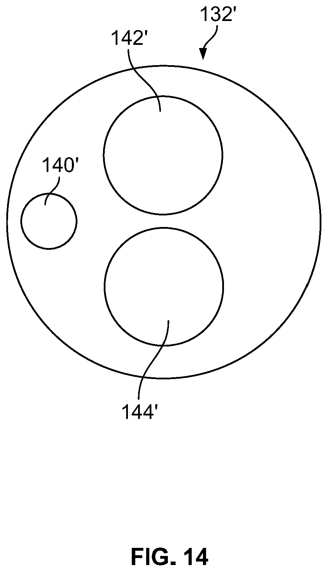

**FIG. 14** is a distal end view of a shaft according to alternative examples.

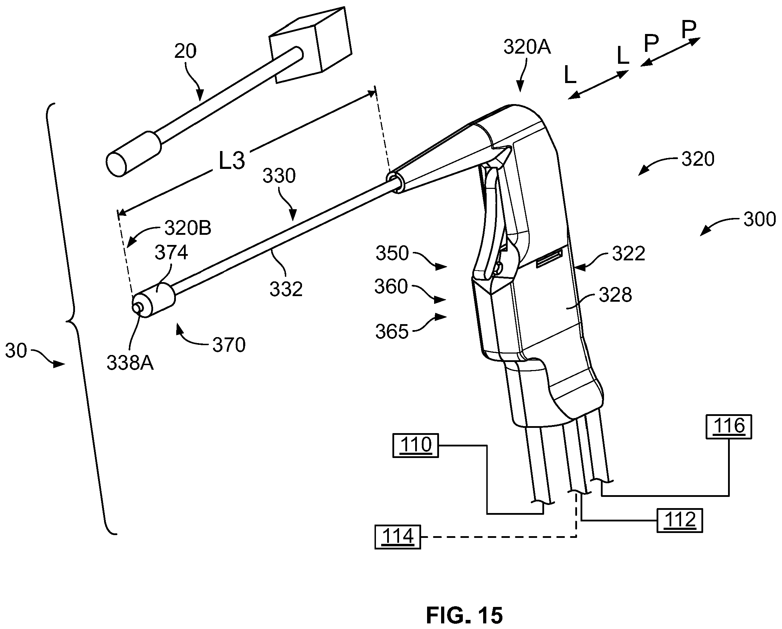

**FIG. 15** is front perspective, schematic view of a sinus treatment system according to further examples.

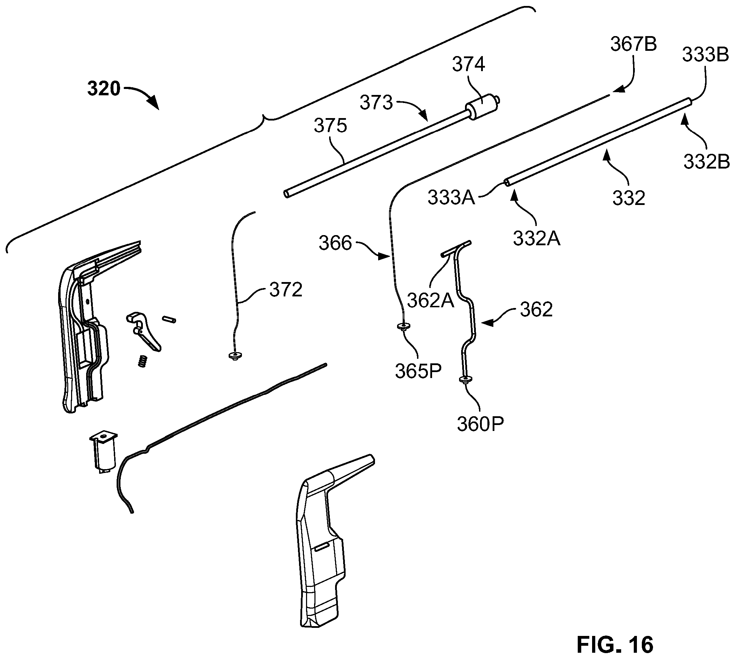

**FIG. 16** is an exploded, rear perspective view of an instrument forming a part of the sinus treatment system of **FIG. 15****.**

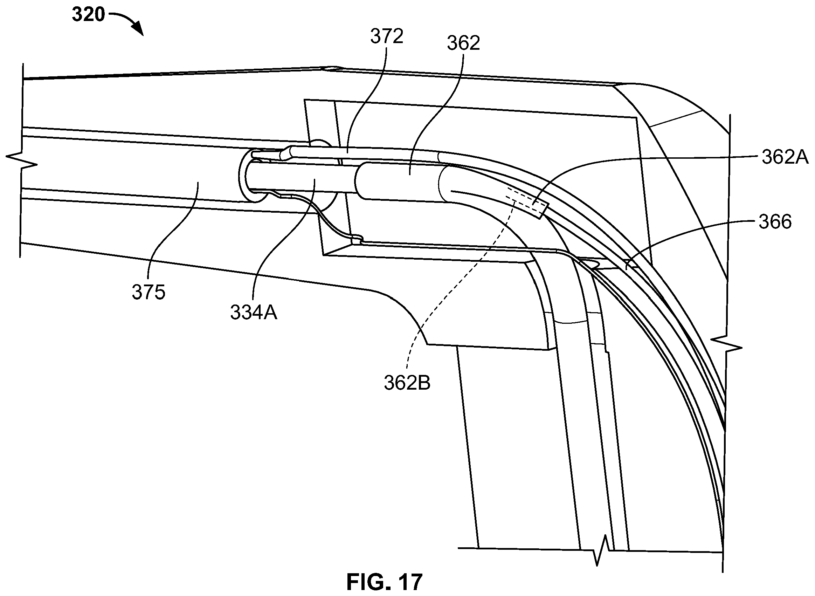

**FIG. 17** is a fragmentary, rear perspective view of the instrument of **FIG. 16****.**

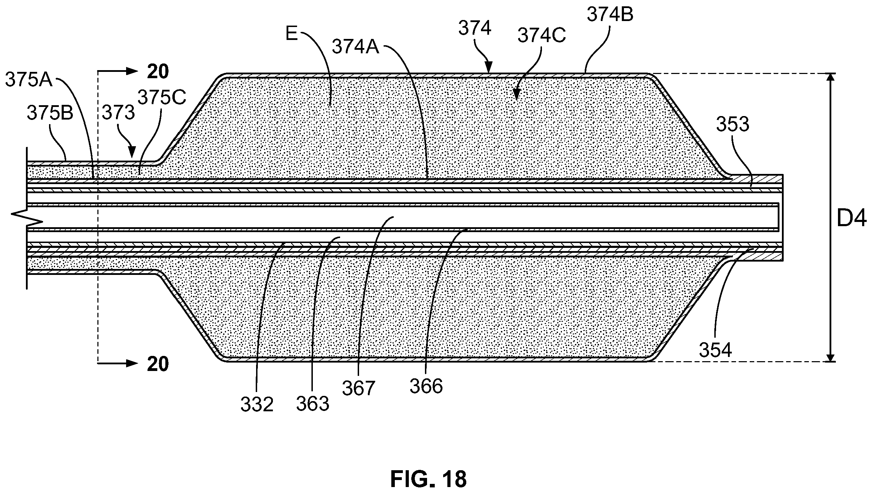

**FIG. 18** is a cross-sectional view of the instrument of **FIG. 16****,** wherein a balloon forming a part of the instrument is in an expanded position.

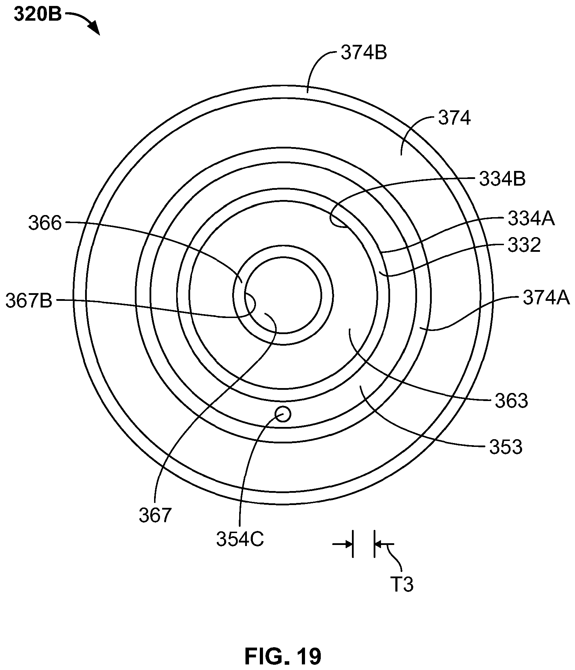

**FIG. 19** is a distal end view of the instrument of **FIG. 16****.**

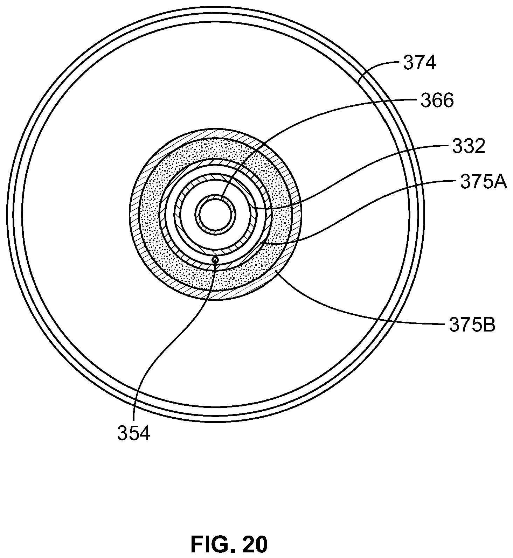

**FIG. 20** is a cross-sectional view of the instrument of **FIG. 16** taken along the line **20-20** of **FIG. 18****.**

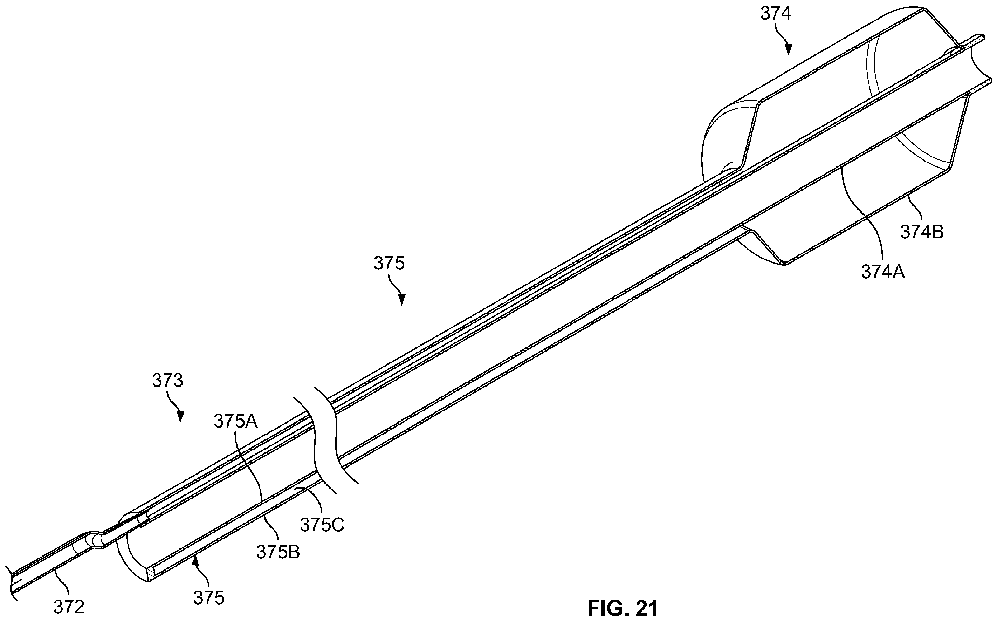

**FIG. 21** is a fragmentary, rear perspective view of a balloon member forming a part of the instrument of **FIG. 16****.**

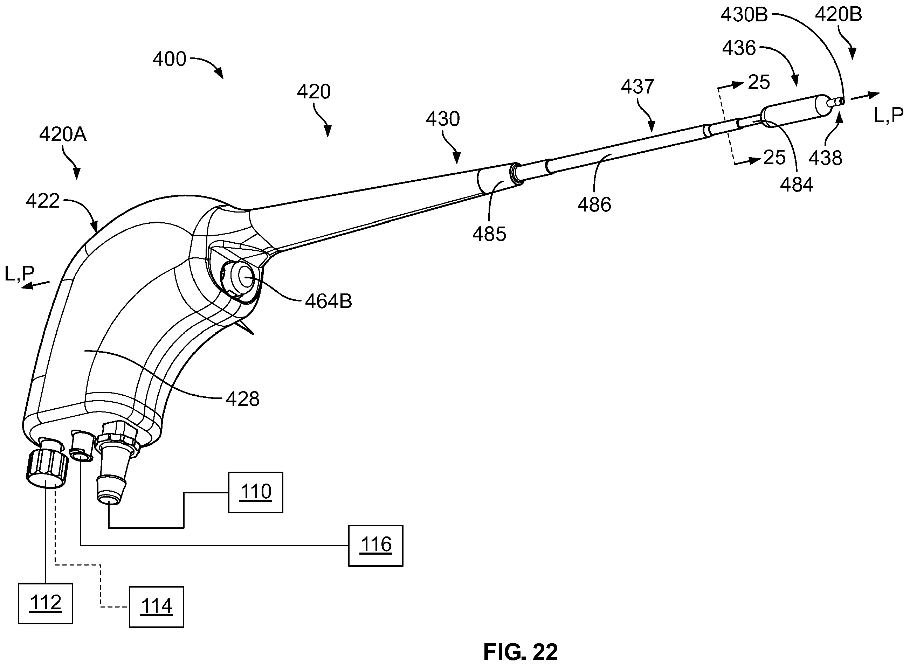

**FIG. 22** is front perspective, schematic view of a sinus treatment system according to embodiments of the invention.

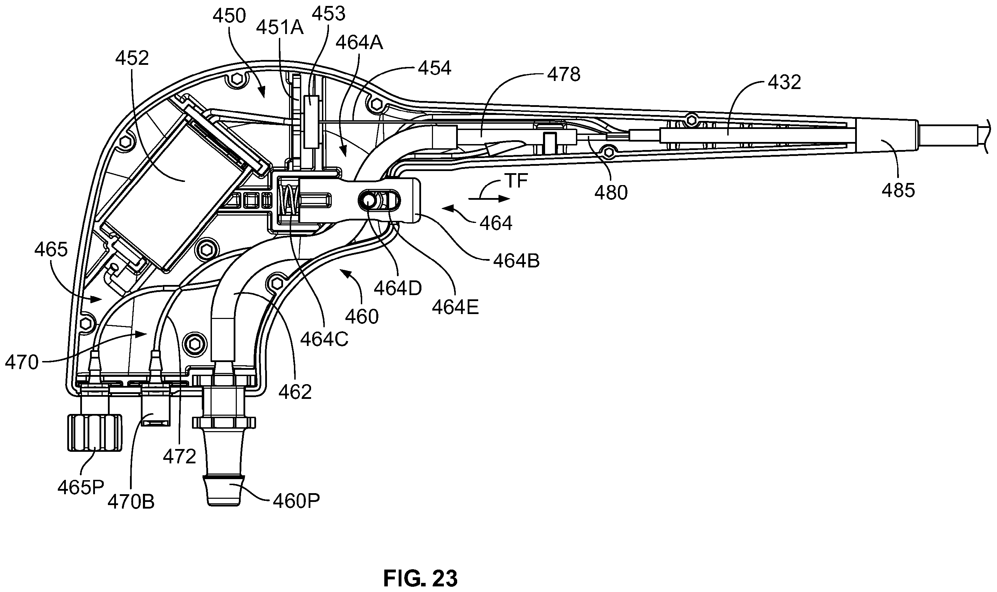

**FIG. 23** is a fragmentary side view of an instrument forming a part of the sinus treatment system of **FIG. 22****.**

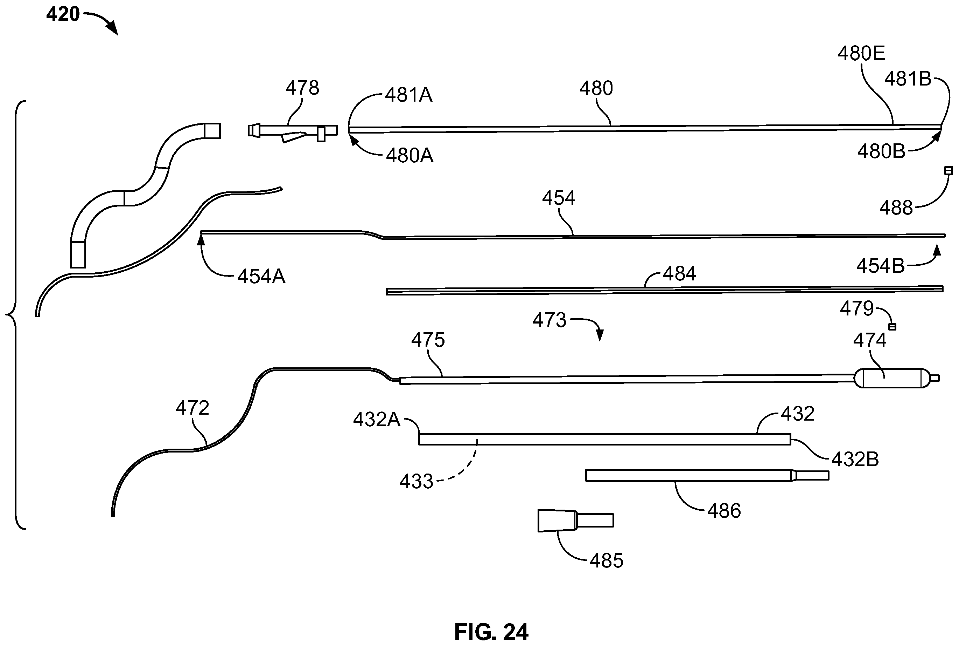

**FIG. 24** is an exploded, fragmentary, side view of the instrument of **FIG. 22****.**

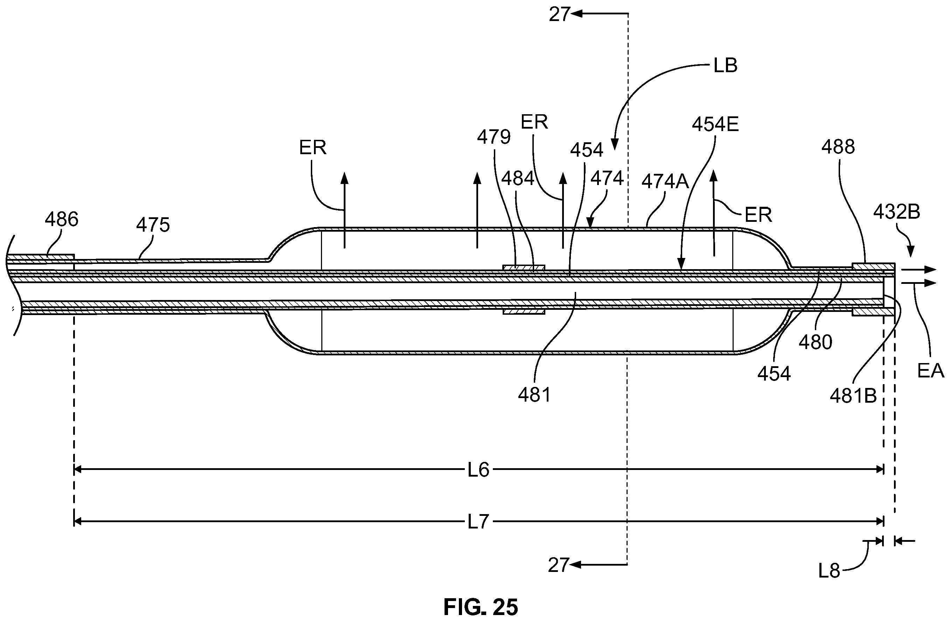

**FIG. 25** is a fragmentary, cross-sectional view of the instrument of **FIG. 22****,** taken along the line **25-25** of **FIG. 22** wherein a dilation balloon forming a part of the instrument is in an expanded position.

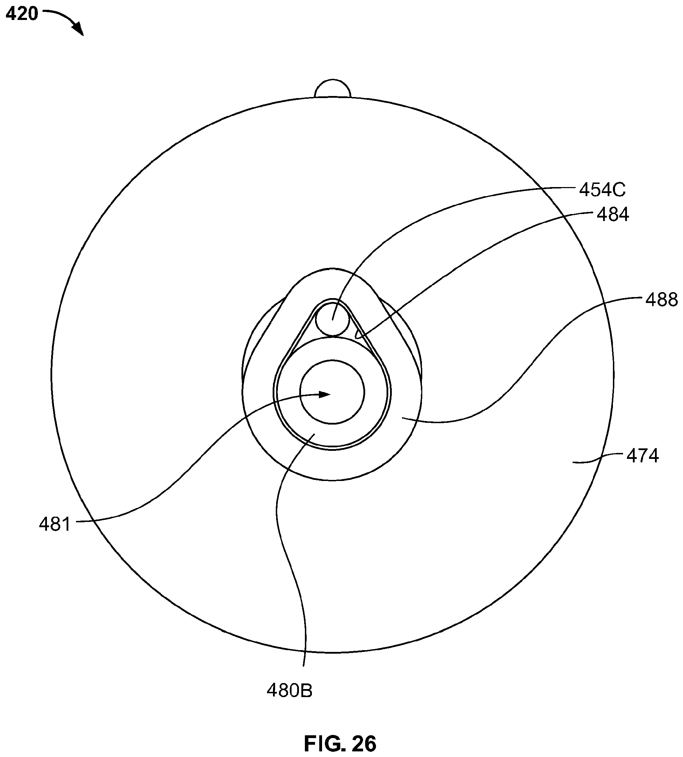

**FIG. 26** is a distal end view of the instrument of **FIG. 22****.**

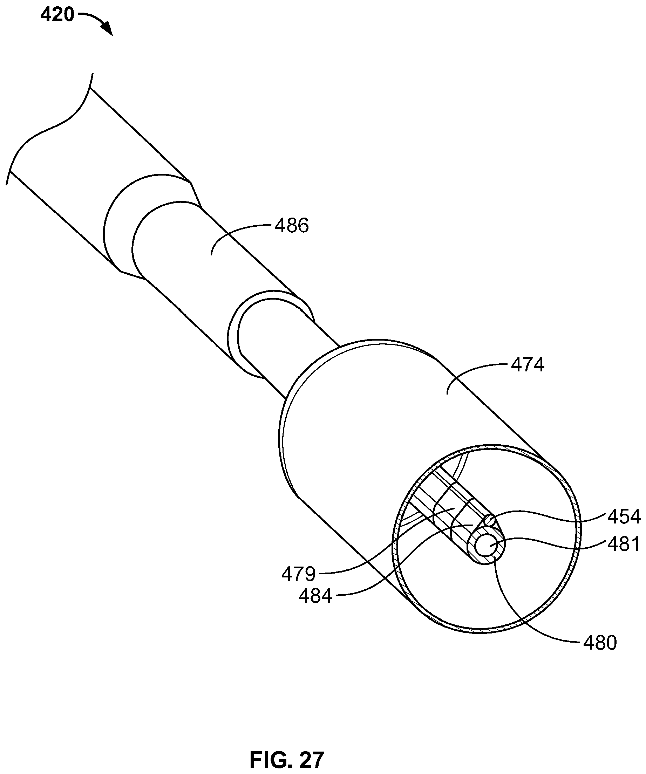

**FIG. 27** is a fragmentary, perspective, cross-sectional view of the instrument of **FIG. 22** taken along the line **27-27** of **FIG. 25****.**

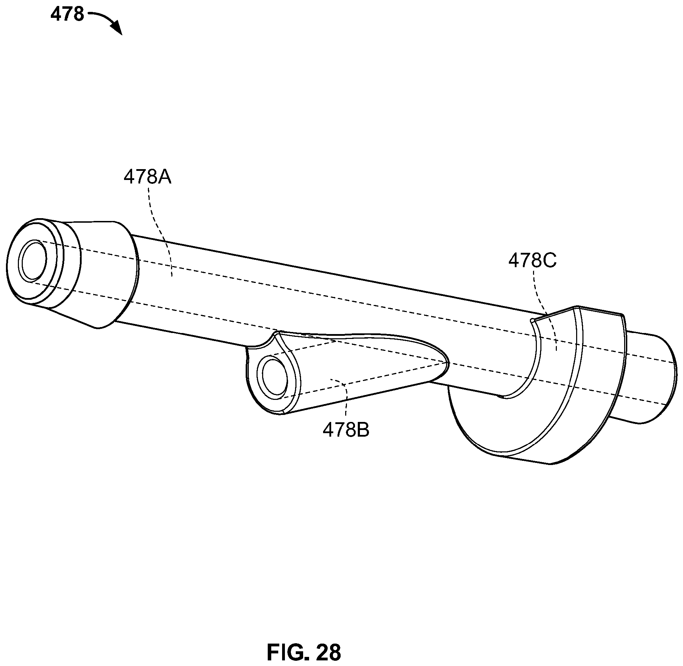

**FIG. 28** is a rear perspective view of a fluid connector manifold forming a part of the instrument of **FIG. 22****.**

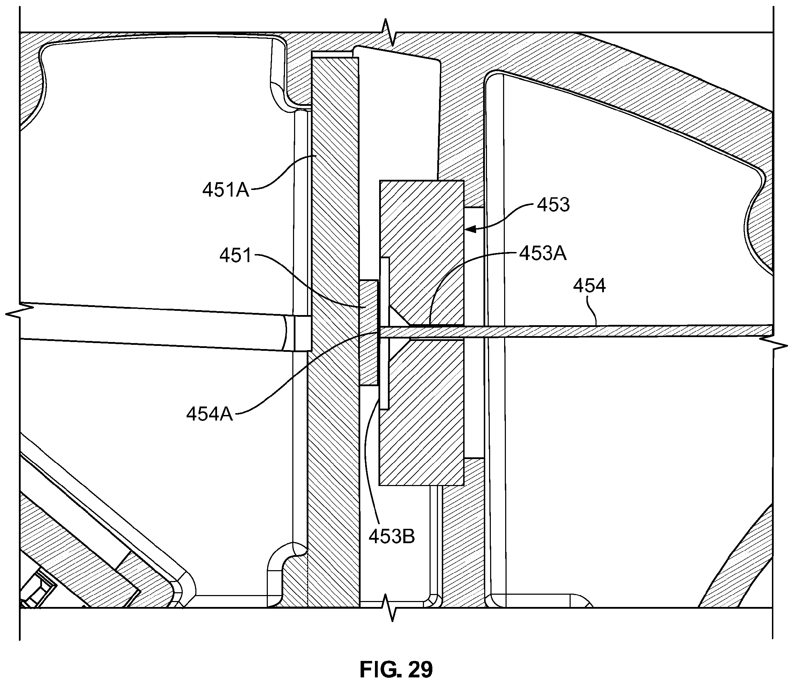

**FIG. 29** is an enlarged, fragmentary, cross-sectional view of the instrument of **FIG. 22****.**

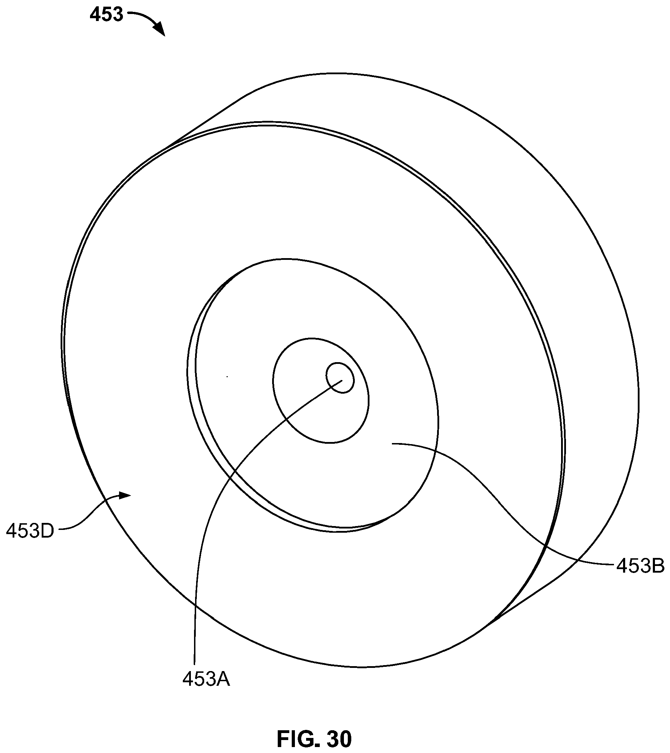

**FIG. 30** is a rear perspective view of a heatsink member forming a part of the instrument of **FIG. 22****.**

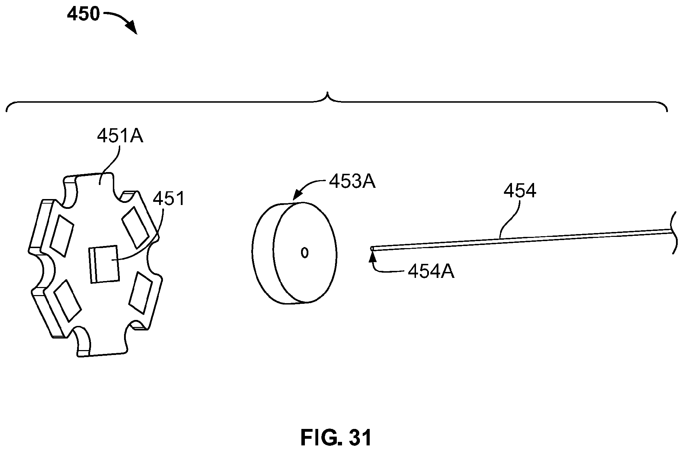

**FIG. 31** is an exploded, front perspective view of the heatsink member and a waveguide and a PCB forming a part of the instrument of **FIG. 22****.**

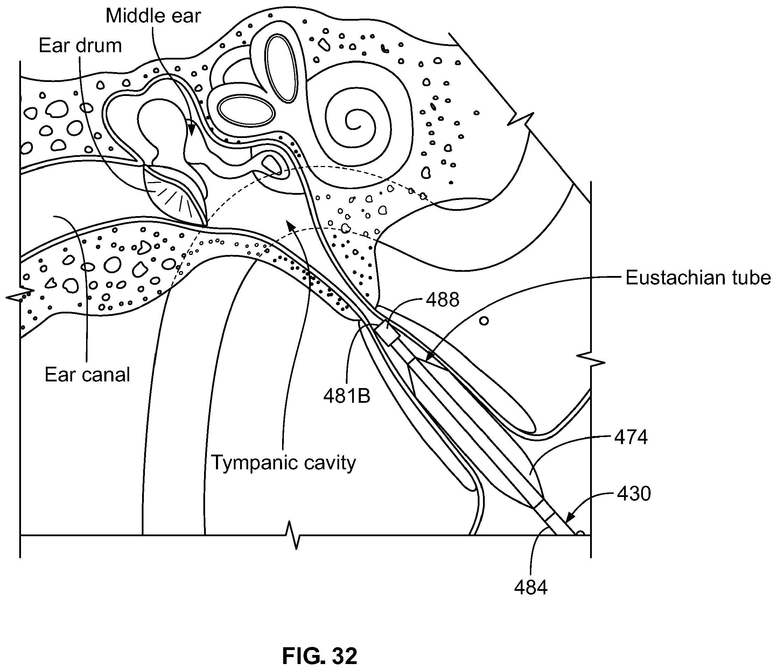

**FIG. 32** illustrates methods using the treatment system of **FIG. 22****.**

### Detailed Description of Embodiments of the Invention

The present invention now will be described more fully hereinafter with reference to the accompanying drawings, in which illustrative embodiments of the invention are shown. In the drawings, the relative sizes of regions or features may be exaggerated for clarity. This invention may, however, be embodied in many different forms and should not be construed as limited to the embodiments set forth herein; rather, these embodiments are provided so that this disclosure will be thorough and complete, and will fully convey the scope of the invention to those skilled in the art.

It will be understood that when an element is referred to as being "coupled" or "connected" to another element, it can be directly coupled or connected to the other element or intervening elements may also be present. In contrast, when an element is referred to as being "directly coupled" or "directly connected" to another element, there are no intervening elements present. Like numbers refer to like elements throughout.

In addition, spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly.

Well-known functions or constructions may not be described in detail for brevity and/or clarity.

As used herein the expression "and/or" includes any and all combinations of one or more of the associated listed items.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

"Treat," "treating" or "treatment of" (and grammatical variations thereof) as used herein refer to any type of treatment that imparts a benefit to a subject and may mean that the severity of a subject's disease, disorder, or condition is reduced, at least partially improved or ameliorated and/or that some alleviation, mitigation or decrease in at least one clinical symptom associated with the disease, disorder, or condition is achieved and/or there is a delay in the progression of the symptom. In some embodiments, the severity of a disease, disorder, or condition associated with a sinus in a subject may be reduced in the subject compared to the severity of the symptom in the absence of a system and/or method of the present invention.

In some embodiments, a therapeutic as described herein may be administered in a treatment effective amount. A "treatment effective amount" as used herein is an amount that is sufficient to treat (as defined herein) a subject. Those skilled in the art will appreciate that the therapeutic effect(s) need not be complete or curative, as long as some benefit is provided to the subject. In some embodiments, a treatment effective amount may be achieved by administering to a subject a medication as described herein, optionally in an irrigation fluid **I,** a supply **114B** of medication **N,** and/or an inflation fluid **E** using a system and/or method of the present invention.

The terms "prevent," "preventing" and "prevention" (and grammatical variations thereof) refer to avoidance, reduction and/or delay of the onset of a symptom associated with a disease, disorder, or condition and/or a reduction in the severity of the onset of a symptom associated with disease, disorder, or condition relative to what would occur in the absence of a system and/or method of the present invention. The prevention can be complete, *e.g.,* the total absence of the symptom. The prevention can also be partial, such that the occurrence of the symptom in the subject and/or the severity of onset is less than what would occur in the absence of a system and/or method of the present invention.

In some embodiments, a therapeutic as described herein may be administered in a prevention effective amount. A "prevention effective amount" as used herein is an amount that is sufficient to prevent (as defined herein) a disease, disorder, or condition and/or a symptom thereof in a subject. Those skilled in the art will appreciate that the level of prevention need not be complete, as long as some benefit is provided to the subject. In some embodiments, a prevention effective amount may be achieved by administering to a subject a medication as described herein, optionally in an irrigation fluid **I,** a supply **114B** of medication **N,** and/or an inflation fluid **E** using a system and/or method of the present invention.

The present invention finds use in both veterinary and medical applications. The term "subject" is used interchangeably herein with the term "patient". Suitable subjects of the present invention include, but are not limited, to mammals of all ages. The term "mammal" as used herein includes, but is not limited to, primates (*e.g.*, simians and humans), non-human primates (*e.g.,* monkeys, baboons, chimpanzees, gorillas), bovines, ovines, caprines, ungulates, porcines, equines, felines, canines, lagomorphs, pinnipeds, rodents (*e.g.,* rats, hamsters, and mice), etc. In some embodiments, the subject is a mammal and in certain embodiments the subject is a human. Human subjects include both males and females and subjects of all ages including fetal, neonatal, infant, juvenile, adolescent, adult, and geriatric subjects.

A system and/or method of the present invention may also used and/or carried out on animal subjects, particularly mammalian subjects such as mice, rats, dogs, cats, livestock and horses for veterinary purposes and/or for drug screening and/or drug development purposes.

As used herein, "monolithic" means an object that is a single, unitary piece formed or composed of a material without joints or seams. Alternatively, a unitary object can be a composition composed of multiple parts or components secured together at joints or seams.

With reference to **FIGS. 1-13F****,** a sinus treatment system **10** according to examples is shown therein. The system **10** includes sinus treatment instrument system **100** and, optionally, an endoscope **20.** The system **10** and the instrument system **100** can be used to conduct procedures (*e.g.*, surgical procedures) on and/or treatment of sinuses. In particular, the system **10** and the instrument system **100** can be used to conduct surgical procedures on and treatment of the paranasal sinuses of a human patient (subject). The system **10** and the instrument system **100** can be used to conduct balloon sinoplasty or balloon dilation of sinus ostia and drainage pathways of a patient. In some embodiments, the system **10** and the instrument system **100** can be used to administer one or more medications to a patient, optionally into a sinus of the patient.

As described herein, the instrument system **100** can be used to execute multiple different operations, namely: an aspiration operation; a fluid delivery operation (irrigation fluid and/or medication); a dilation operation; and an illumination operation. Each of these operations can be executed using the instrument system **100** simultaneously (*i.e.,* at the same time) or independently, in any desired combination. By "independently", it is meant that the operation can be selectively conducted while selectively not conducting another of the operations.

The instrument subsystem **100** includes a handheld instrument **120,** a suction (negative pressure or vacuum) source **110,** an irrigation fluid source **112,** a medication source **114,** and an inflation fluid source **116.**

The suction source **110** may include a pump or compressor such as an electric pump or compressor, a syringe, or any other suitable device for generating a negative pressure (vacuum) to enable aspiration. In some embodiments, the suction source **110** generates and maintains a substantially constant negative pressure. The pressure level may be set by the operator.

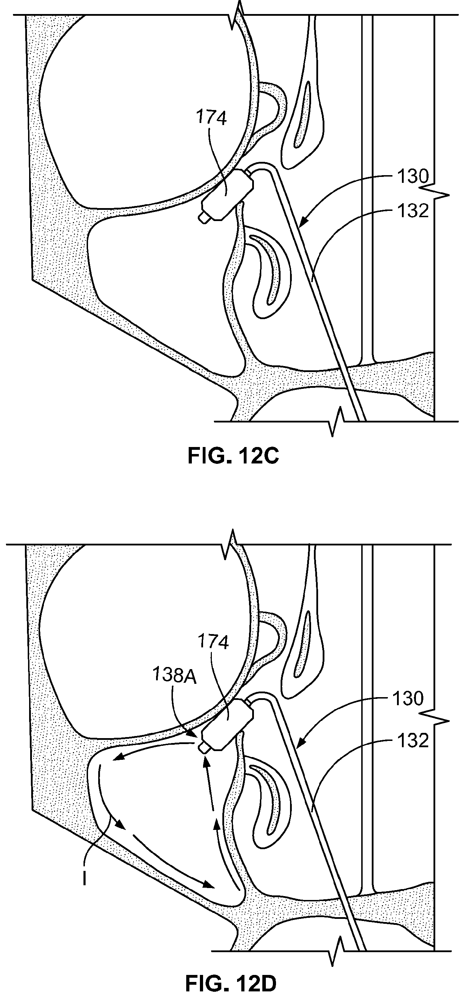

The irrigation fluid source **112** may include a pump **112A** and a supply **112B** of irrigation fluid **I (****FIG. 12D****).** The irrigation fluid **I** may be any suitable fluid and, in some embodiments, is an irrigation liquid. In some embodiments, the irrigation fluid **I** is water or saline. In some embodiments, the irrigation fluid source **112** is a syringe containing the irrigation fluid supply **112B.** The irrigation syringe may be a hand-operated syringe. In some embodiments, the irrigation fluid **I** comprises a medication.

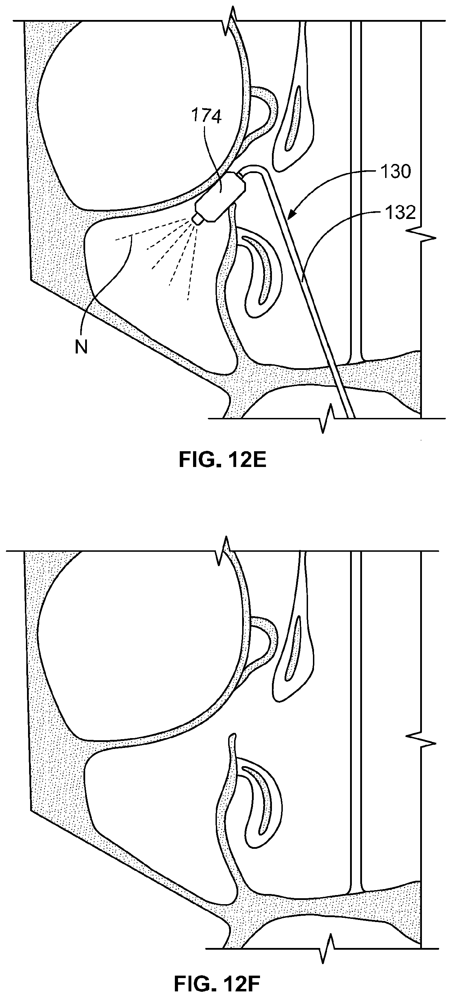

The medication source **114** may include a pump **114A** and a supply **114B** of medication **N (****FIG. 12E****).** The medication **N** may be any suitable flowable medication and, in some embodiments, is a liquid. In some embodiments, the medication is a medication fluid. In some embodiments, the medication source **114** is a syringe containing the medication supply **114B.** The medication syringe may be a hand-operated syringe.

The inflation fluid source **116** may include a pump **116A** and a supply **116B** of inflation fluid **E (****FIG. 1****).** The inflation fluid may be any suitable fluid and, in some embodiments, is an inflation liquid. In some embodiments, the inflation fluid **E** is water or saline. In some embodiments, the inflation fluid source **116** is a syringe containing the inflation fluid supply **116B.** The inflation syringe may be a hand-operated syringe. In some embodiments, an inflation fluid **E** comprises a medication.

"Medication" as used herein refers to a therapeutic and/or diagnostic agent. One or more medication(s) (*e.g*., one or more therapeutic(s) and/or one or more diagnostic agent(s)) may be present in the irrigation fluid **I,** the supply **114B,** and/or the inflation fluid **E.** A medication may be in any suitable form. In some embodiments, a medication is present in a solution (*e.g.*, an aqueous solution), suspension, or emulsion. In some embodiments, the medication source **114** is not present when a medication is not needed or is not to be administered and/or when a medication is present in the irrigation fluid **I** and/or inflation fluid **E.** "Therapeutic" as used herein refers to any chemical and/or biological compound and/or agent that can be used to treat and/or prevent a disease, disorder, or condition and/or a symptom thereof in a subject. Therapeutics include, but are not limited to, antibiotics, antiviral agents, antiparasitic agents, antifungal agents, anti-inflammatory agents, decongestants, mucous thinning agents, and/or steroids. "Diagnostic agent" as used herein refers to any substance that aids in the diagnosis of a disease, disorder or condition in a subject and/or that aids in the delivery of a therapeutic and/or device or component thereof to a subject. Diagnostic agents include, but are not limited to, contrast agents and/or imaging agents. In some embodiments, a medication may be one or more diagnostic or therapeutic substances such as those as described in U.S. Patent No. 7,361,168 and optionally a method of administering and/or delivering a medication to a subject may be as described in U.S. Patent No. 7361168.

A medication may be administered and/or delivered to a subject using any means and/or method known to those of skill in the art. In some embodiments, a medication may be administered and/or delivered to a subject via a fluid (*e.g.,* a solution (*e.g.,* an aqueous solution), suspension, emulsion, etc.) comprising the medication. The fluid may be an irrigation fluid **I,** a fluid provided in supply **114B,** and/or an inflation fluid **E.** In some embodiments, the inflation fluid **E** comprises a medication and the inflation fluid **E** is administered to a subject through a balloon **174, 374** (discussed below). The balloon **174, 374** may comprise one or more pore(s) and the inflation fluid **E** comprising at least one medication may flow out of the one or more pore(s) of the balloon **174, 374** and optionally into a sinus. In some embodiments, the one or more pore(s) may open and/or expand when the inflation fluid **E** is filling and/or has filled the balloon **174, 374,** optionally to a given volume and/or pressure, and upon opening and/or expanding the inflation fluid **E** flows, leaks, weeps and/or the like from the one or more pore(s). In some embodiments, a medication is provided on the balloon **174, 374** such as, for example, in the form of a coating on the balloon **174, 374.** The coating and/or medication may be administered to a subject upon contact with the coating such as, *e.g.,* upon the balloon **174, 374** expanding and/or contacting a tissue and/or a nasal and/or sinus surface of the subject. In some embodiments, the coating and/or medication are administered to a subject upon the balloon **174, 374** blocking and/or plugging a sinus passage of the subject. In some embodiments, the system **10** and instrument **120** may administer and/or deliver an implant comprising a medication. The implant may be in the form of a coil, stent, spike, wire, mesh, patch, and/or the like. The system **10** and instrument **120** may attach the implant to a tissue and/or a nasal and/or sinus surface of a subject. In some embodiments, the system **10** and instrument **120** may embed the implant into a tissue and/or a nasal and/or sinus surface of a subject. In some embodiments, the implant is attached and/or embedded upon the balloon **174, 374** expanding and/or contacting a tissue and/or a nasal and/or sinus surface of the subject, optionally upon the balloon **174, 374** blocking and/or plugging a sinus passage of the subject. The implant may be biodegradable and/or bioabsorbable. The implant may comprise a medication and optionally a carrier material (*e.g.*, a polymer). In some embodiments, an implant may be one as described in U.S. Patent No. 7,361,168, and optionally may be delivered as described in U.S. Patent No. 7361168.

The instrument **120** is a balloon dilation catheter device that includes functional features and mechanisms in addition to the dilation functionality. The instrument **120** includes a handheld unit or base **122,** a probe (or guide member or extension assembly) **130,** a lighting system **150,** an aspiration system **160,** a delivery system **165,** a dilation system **170,** and a connector fitting **178.** The instrument **120** has a proximal end **120A** and a distal end **120B** defining a primary or longitudinal axis **L-L.**

Base **122** has a main axis **M-M** substantially parallel to the instrument longitudinal axis **L-L.**

The base **122** includes a housing **124** including opposed parts **124B** defining an internal cavity **124A.** Suitable features such as channels, cavities, posts and the like may be provided in the base **122** and the cavity **124A** to receive, position and secure the various components housed in the base **122,** as described herein.

The housing **122** has a main section **126** extending from a proximal end **126A** to a distal end **126B** parallel to the main axis **M-M.** A distal port **127** is defined in the distal end **126B.**

The base **122** further includes a handle section **128** that defines a handle axis **H-H** transverse to the main axis **M-M.** The handle section **128** has an inner end **128B** that merges with the main section **126** and an opposing outer end **128A.** In some embodiments, the handle axis **H-H** forms an angle **A1 (****FIG. 3****)** with the main axis **M-M** in the range of from about 50 to 130 degrees and, in some embodiments, in the range of from about 70 to 110 degrees. In some embodiments, the handle axis **H-H** is substantially perpendicular to the main axis **M-M** and the instrument longitudinal axis **L-L.**

The base **122** is ergonomically shaped or contoured to have a rear handle face **129A,** a top face **129B,** an upper shoulder **129C,** a lower front handle face **129D,** an upper front handle face **129E,** and a middle front handle face **129F.** The face **129A** is located rearward of the top face **129B** and connected thereto by the rounded transition or shoulder face **129C.** The middle front handle face **129F** projects forwardly beyond the lower front handle face **129D** and the upper front handle face **129E.**

The probe **130** includes an elongate shaft **132** defining a longitudinal probe axis **P-P** extending from a proximal end **132A** to a distal end **132B.** The probe axis **P-P** is substantially parallel to the instrument axis **L-L** and the main axis **M-M.** The shaft **132** includes a dilator section **136,** an intermediate section **137,** and a tip section **138.** The tip section **138** terminates at a tip **138A.**

The shaft **132** has an outer surface **134A.** In some embodiments, the outer surface **134A** is substantially cylindrical. In some embodiments, the outer surface **134A** is substantially conical or frusto-conical. In some embodiments, the outer surface **134A** is substantially elliptical in lateral cross-section and, in some embodiments, is substantially circular in lateral cross-section.

The shaft **132** may be formed of any suitable material(s). In some embodiments, the shaft **132** is formed of a stainless steel hypotube.

In some embodiments, the shaft **132** is unitary. In some embodiments, the shaft **132** is unitarily formed. In some embodiments, the shaft **132** is monolithic. The shaft **132** may be molded (*e.g.,* injection molded) or extruded.

In some embodiments, the shaft **132** is formed of a rigid but malleable material. This permits the shaft **132** to be deliberately bent into a new shape or configuration in response to application of a sufficient bending force, and to retain the original or new shape or configuration when a lesser force is applied to the shaft **132.** In some embodiments, the required bending force is greater than any force the shaft **132** is expected or intended to experience in service during the surgical procedure (*i.e.,* during navigation or use of the probe **120** within the anatomy of the patient). The malleability of the shaft **132** may enable the user to bend the shaft **132** into a desired angle or curvature to achieve proper positioning for the balloon **174** and the distal end tip **138A** in the sinus ostia or sinus drainage pathways.

In some embodiments, the shaft **132** is pre-shaped to have a curved distal portion (*e.g.,* as shown in **FIGS. 12A-12E****).** The curved distal portion may be configured to match with the frontal sinus outflow tract or frontal recess.

In some embodiments, the distal portion the shaft **132** is formed right to the distal end **132B** and may have a radius of curvature in the range of from about 0.25 inch to about 1.5 inch and, in some embodiments, from about 0.75 to about 1.25 inch.

The shaft **132** extends a predetermined or prescribed distance or length **L1 (****FIG. 3****)** from the base **122** to the distal end **132B.** In some embodiments, the length **L1** is in the range of from about 4 to 12 inches and, in some embodiments, from about 8 to 10 inches.

In some embodiments, the shaft **132** has an outer diameter **D1 (****FIG. 6****)** in the range of from about 1 to 5 mm and, in some embodiments, from about 1.5 to 3.3 mm.

In some embodiments, the shaft **132** has a nominal wall thickness **T1 (****FIG. 6****)** in the range of from about 0.001 to 0.030 inch and, in some embodiments, from about 0.005 to 0.020 inch.

In some embodiments, the distal tip **138A** has an outer diameter in the range of from about 0.5 to 5 mm and, in some embodiments, from about 1 to 3.3 mm.

In some embodiments, the edges of the tip **138A** are rounded or smooth to prevent or reduce trauma to the mucosa of the sinuses.

A lighting lumen **140,** an aspiration lumen **142,** and a delivery lumen **144** are defined in the shaft **132.** The each of the lumens **140, 142, 144** is contained or located within the boundary defined by the shaft outer surface **134A.** The lumens **140, 142, 144** are elongate and extend radially or laterally side-by-side relative to one another along the axis **P-P.** In some embodiments, the lumens **140, 142, 144** extend substantially parallel to one another along the axis **P-P.**

The lighting lumen **140** terminates at a proximal opening **140A** at the end **132A** and at a distal opening **140B** at the end **132B.** The aspiration lumen **142** terminates at a proximal opening **142A** at the end **132A** and at a distal opening or aspiration inlet port **142B** at the end **132B.** The delivery lumen **144** terminates at a proximal opening **144A** at the end **132A** and at a distal opening or fluid delivery outlet port **144B** at the end **132B.** The distal openings **140B, 142B, 144B** are formed in the tip **138A.**

In some embodiments, each lumen **140, 142, 144** is substantially uniform in size and cross-sectional area throughout its length and including its proximal and distal end openings. In other embodiments, one or more of the lumens **140, 142, 144** may be nonuniform.

In some embodiments and as shown in **FIG. 10****,** one or more of the lumens **140, 142, 144** may have a different cross-section shape and/or area than another of the lumens **140, 142, 144.** In some embodiments and as shown in **FIG. 10****,** one or more of the lumens **140, 142, 144** may have an irregular or asymmetric shape (*e.g*., non-elliptical and non-annular) that more closely optimizes the use of the available area within the shaft **132.**

With reference to **FIG. 10****,** in some embodiments the cross-sectional area of the aspiration lumen **142** is greater than the cross-sectional area of the delivery lumen **144.** In some embodiments, the cross-sectional area of the aspiration lumen **142** is at least 1.5 times the cross-sectional area of the delivery lumen **144.** In some embodiments, the cross-sectional area of the aspiration lumen **142** is in the range of from about 2 to 4 times the cross-sectional area of the delivery lumen **144.**

In some embodiments, the cross-sectional area of the aspiration lumen **142** is greater than the cross-sectional area of the lighting lumen **140.** In some embodiments, the cross-sectional area of the aspiration lumen **142** is at least 15 times the cross-sectional area of the lighting lumen **140.** In some embodiments, the cross-sectional area of the aspiration lumen **142** is in the range of from about 8 to 40 times the cross-sectional area of the lighting lumen **140.**

In some embodiments, the cross-sectional area of the delivery lumen **144** is greater than the cross-sectional area of the lighting lumen **140.** In some embodiments, the cross-sectional area of the delivery lumen **144** is at least 8 times the cross-sectional area of the lighting lumen **140.** In some embodiments, the cross-sectional area of the delivery lumen **144** is in the range of from about 4 to 10 times the cross-sectional area of the lighting lumen **140.**

In some embodiments, the cross-sectional area of the aspiration lumen **142** is in the range of from about 0.28 to 1.76 mm².

In some embodiments, the cross-sectional area of the delivery lumen **144** is in the range of from about 0.07 to 0.8 mm².

In some embodiments, the cross-sectional area of the lighting lumen **140** is in the range of from about 0.007 to 0.2 mm². In some embodiments, the cross-sectional area of the lighting lumen **140** is substantially the same as the outer diameter of the waveguide **154** (including the sheath).

In some embodiments, the cross-sectional area of the through passage or lumen of the inflation conduit **172** is less than the cross-sectional area of the aspiration lumen **142** and, in some embodiments, less than the cross-sectional areas of the delivery lumen **144** and the lighting lumen **140.**

The connector fitting **178 (****FIGS. 2****,** **5** and **11****)** includes an aspiration channel **178A** and a delivery channel **178B** each extending fully therethrough. The distal end of the fitting **178** is mated and secured to the proximal end **132A** of the shaft **132** such that the distal opening of the aspiration channel **178A** is fluidly connected to the aspiration lumen **142,** and the distal opening of the delivery channel **178B** is fluidly connected to the delivery lumen **144.** The fitting **178** may be formed of any suitable material (*e.g*., metal) and may be secured to the proximal end of the shaft **132** by any suitable technique (*e.g*., welding or adhesive).

The integral lighting system **150** includes a light source **151,** an internal battery **152,** and a waveguide **154.** In some embodiments, the light source **151,** the battery **152** and a battery contact spring are mounted in the cavity **124A** of the base **122.** In some embodiments, the light source **151** includes a light emitting diode (LED) and may include an associated printed circuit board (PCB).

In some embodiments, the waveguide **154** is an optical fiber (*e.g.,* a polymeric or glass optical fiber). The waveguide **154** may be an optical fiber including a core and a surrounding cladding and may be configured to provide total internal reflection. The waveguide **154** may be surrounded by a sheath to protect the waveguide and prevent light loss. A light emitting guide wire such as that disclosed in U.S. Patent Application Publication No. 2007/0249896 can be used as the waveguide **154.**

The waveguide **154** extends from a proximal end **154A** in the base **122** and proximate the light source **151,** through the opening **140A,** through the lighting lumen **140,** and to a distal end **154B** adjacent the distal end **132B.** The waveguide **154** has an end face **154C** at the distal end **154B,** from which light is emitted. The end face **154C** may be polished. In some embodiments, the waveguide distal end **154B** is located substantially flush with the distal end **132B.** In other embodiments, the waveguide distal end **154B** extends outwardly beyond the distal end **132B** a distance in the range of from about 1 to 5 cm.

As shown in **FIGS. 1** and **4****,** an electrically insulating switch tab **151A** electrically disconnects a terminal of the battery **152** from the lighting circuit until it is desired to operate the light source **151.** In use, the operator removes the tab **151A,** thereby actuating the light source **151** to generate light that is transmitted through the waveguide **154** and emitted from the distal end **154B.** Alternatively, the lighting system **150** may include a light switch to turn the light source on and off.

In some embodiments, the instrument **120** may include an external power supply (*e.g.*, connected to the instrument **120** by a power cord) in place of or in addition to the battery **152.**

The aspiration system **160** includes a conduit **162,** a suction port **160P,** and an aspiration controller **164.** The suction port **160P** is provided in the outer end of the handle **128.** The conduit **162** extends through the handle **128.** A proximal end of the conduit **162** is fluidly connected to the suction port **160P.** The distal end of the conduit **162** is fluidly coupled to the aspiration channel **178A** of the connector fitting **178,** and is thereby fluidly connected to the proximal opening **142A** of the aspiration lumen **142.** The suction source **110** is fluidly connected to the suction port **160P** by a conduit **160T.** The suction port **160P** may be provided with a connector or coupling (such as a Luer lock) to fluidly connect the conduit **160T** from the suction source **110** to the port **160P.**

The aspiration controller **164** includes a valve **164A** in the base **122,** an integral trigger **164B** attached to the base **122,** and a return spring **164C.** The trigger **164B** is operable to selective allow and prevent fluid flow between the lumen **142** and the suction source **110** through the conduit **162.**

In some embodiments and as shown, the valve **164A** is a pinch valve. The trigger **164B** is positioned proximate the handle **128** such that the trigger **164B** can be conveniently pulled using the operator's finger. A portion **164D** of the trigger **164B** serves as a pinching feature that compresses and closes the conduit **162** when the trigger **164B** is not depressed. When the trigger **164B** is depressed, the conduit **162** is opened. Upon release, the trigger **164B** is urged back to the closed position by the spring **164C.** The trigger **164B** thereby operates as a hand-actuated valve **164A** that can be selectively operated by the user to open and close the fluid pathway between the suction source **110** and the lumen **142.**

In alternative embodiments, the trigger **164B** is configured such that its operation is reversed. In that case, the conduit **162** is open by default. When the trigger **164B** is depressed, the conduit **162** is closed. Upon release, the trigger **164B** is urged back to the open position by the spring **164C.**

The delivery system **165** includes a conduit **166,** a delivery port **165P,** and a delivery controller **168** (for irrigation fluid) or a delivery controller **169** (for medication). The delivery port **165P** is provided in the outer end of the handle **128.** The conduit **166** extends through the handle **128.** A proximal end of the conduit **166** is fluidly connected to the delivery port **165P.** The distal end of the conduit **166** is fluidly coupled to the delivery channel **178B** of the connector fitting **178,** and is thereby fluidly connected to the proximal opening **144A** of the delivery lumen **144.** The delivery source **112** is fluidly connected to the delivery port **165P** by a conduit **165T.** The delivery port **165P** may be provided with a connector or coupling (such as a Luer lock) to fluidly connect a conduit **165T** from the irrigation fluid source **112** to the port **165P.**

The delivery controller **168** is operable to control actuation of the delivery source **112** and/or to selectively allow and prevent fluid flow through the conduits **166, 165T** to the delivery lumen **144.** In some embodiments, the controller **168** is offboard from the instrument **120.** In some embodiments, the delivery source **112** is embodied in a syringe that includes a reservoir (supply **112B)** and a pump mechanism (pump **112A** and controller **168).** In some embodiments, the syringe is a hand operated syringe. The medication delivery controller **169** can be likewise constructed and operated to control delivery of medication from the source **114.**

The dilation system **170** includes a dilator mechanism **171** (in the form of a balloon **174),** a conduit **172,** an inflation port **170P,** and a dilation controller **176.** The balloon **174** is mounted proximate the distal end **132B** of the shaft **132.** The dilation system **170** is configured to selectively inflate and deflate the balloon **174** on demand by the operator.

The inflation port **170P** is provided in the outer end of the handle **128.** The conduit **172** extends through the handle **128.** A proximal end of the conduit **172** is fluidly connected to the inflation port **170P.** The inflation source **116** is fluidly connected to the inflation port **170P** by a conduit **170T.** The inflation port **170P** may be provided with a connector or coupling (such as a Luer lock) to fluidly connect a conduit **170T** from the inflation source **116** to the port **170P.**

A connecting section of the conduit **172** extends along the outer surface **134A** of the shaft **132** from the handle **128** to the balloon **174.** The distal end of the conduit **172** extends into the balloon **174** through a port **175** defined between the balloon **174** and the outer diameter of the shaft **132.** The connecting section may be secured to the shaft **132** by tape, adhesive, welding, or any other suitable method.

The balloon **174** includes sealed ends **174B,** a main section **174A** therebetween, and an outer surface **174C.** In some embodiments, the balloon **174** is mounted directly on the shaft **132** so as to form a fluidic seal between the two components. The balloon **174** may be bonded to the shaft **132** using a weld, adhesive, or the like. Alternatively, the balloon **174** may be secured to the shaft **132** using a mechanical connection. The balloon **174** and the shaft **132** define a sealed balloon inflation chamber **177** therebetween.

The balloon **174** is pliable and expandable when inflated. The dilation system **170** can be operated to place the balloon **174** alternatively in at least a first, relatively radially non-expanded configuration (herein, the non-expanded position) and a second, radially expanded configuration (herein, the expanded configuration). In the expanded configuration, the outer diameter **D2 (****FIG. 8****)** of the balloon **174** is larger than in the deflated configuration.

In some embodiments and as will be appreciated from the description herein, the dilation system **170** can be operated to place the balloon **174** in a range of different expanded configurations. That is, the balloon **174** can be forced to assume one of a plurality of different expanded configurations each having a different outer diameter **D2.**

In some embodiments and as will be appreciated from the description herein, the non-expanded configuration of the balloon **174** may not be a fully non-expanded or deflated configuration. That is, the outer diameter of the balloon **174** may be greater than is smallest possible diameter in the non-expanded configuration.

In some embodiments, the main section **174A** takes on a cylindrical shape when the balloon **174** is substantially fully inflated (*e.g.,* as shown in **FIGS. 6****,** **8** and **9****).** In some embodiments, the end sections **174B** take on a frusto-conical shape when the balloon **174** is substantially fully inflated. However, other shapes may be utilized depending upon the target anatomy.

In some embodiments, the balloon **174** when fully inflated has an outer diameter **D2** in the range of about 3 mm to about 9 mm.

In some embodiments, the balloon **174** when fully inflated has a fully inflated length **L2** in the range of about **10** mm to 25 mm.

The balloon **174** may be formed of any suitable material. In some embodiments, the balloon **174** is formed of high strength but flexible polymeric material such as a polyamide (*e.g*., nylon), PEBAX or the like. The balloon **174** may be "blow molded" to a relatively thin wall thickness, and capable of holding relatively high pressures from about 6 atmospheres to about 28 atmospheres of inflation pressure.

The dilation controller **176** is operable to control actuation of the inflation fluid source **116** to selectively force inflation fluid **E** flow through the conduits **172, 170T** to the balloon **174** to expand the balloon **174** and, alternatively, permit (or forcibly draw) fluid flow from the balloon **174,** through the conduits **172, 170T** to deflate the balloon **174.**

In some embodiments, the dilation controller **176** is offboard from the instrument **120.** In some embodiments, the inflation fluid source **116** is embodied in a syringe that includes a reservoir (supply **116B)** and a pump mechanism (pump **116A** and dilation controller **176).** In some embodiments, the syringe is a hand operated syringe. In some embodiments, the dilation controller **176** includes a closed loop fluid pump system. One exemplary inflation device that may be used to selectively inflate the balloon **174** is described in U.S. Published Patent Application No. 2010/0211007. Other inflation devices may also be used.

The sinus treatment system **10** and the instrument **120** may be used as follows in accordance with methods of the invention.

The system **10** can be set up by connecting the conduits **160T, 165T, 170T** to the ports **160P, 165P, 170P,** respectively. The ports **160P, 165P, 170P** may include disconnectable connectors. If an endoscope is to be used, the endoscope **20** may be set up as needed. If an irrigation step is to be executed, the conduit **165T** is connected to the irrigation fluid source **112.** If a medication delivery step is to be executed, the conduit **165T** is connected to the medication source **114.**

Generally, the lighting system **150** is activated by removing the tab **151A** (or operating a light actuation switch on the instrument **120,** if any), thereby permitting the battery **152** to power the LED **151.** The light emitted by the LED **151** propagates through the waveguide **154** and is emitted from the waveguide **154** through the end face **154C.** The illumination function of the instrument system **100** is thereby selected and actuated and can be used to execute on an illumination operation wherein light is emitted from the waveguide end face **154C** (*e.g.*, trans-illumination).

The aspiration system **160** is actuated and deactuated using the trigger **164B** as described above. When the trigger **164B** is depressed, the suction source **110** will generate a negative pressure (vacuum) in the aspiration lumen **142** and at the opening **142B.** The negative pressure will induce an aspiration flow into the aspiration lumen **142** through the inlet port **142B,** and out of the instrument **120** through the conduits **162, 160T.** The aspiration function of the instrument system **100** is thereby selected and actuated and can be used to execute an aspiration operation. When the trigger **164B** is released, the negative pressure and aspiration flow are terminated.

The delivery system **165** is actuated and deactuated using the irrigation controller **168** or the medication delivery controller **169,** whichever is connected to the port **165P.** The fluid delivery function of the instrument system **100** is thereby selected and actuated and can be used to execute a fluid delivery option. The fluid delivery operation can be an irrigation fluid delivery operation and/or a medication delivery operation.

If the irrigation system **112** is connected, the irrigation controller **168** is actuated to force a flow of the irrigation fluid **I** through the conduits **166, 165T** and the delivery lumen **144,** and out of the probe **130** through the outlet port **144A.**

If the medication delivery system **114** is connected, the medication delivery controller **169** is actuated to force a flow of the medication **N** through the conduits **166, 165T** and the delivery lumen **144,** and out of the probe **130** through the outlet port **144A.**

The dilation system **170** is actuated and deactuated using the dilation controller **176.** In an inflation operation or step, the dilation controller **176** is operated to force a flow of the inflation fluid **E** through the conduits **172, 170T** and into the balloon cavity **177,** pressurize the inflation fluid **E** in the balloon cavity **177,** and maintain the pressure of the inflation fluid **E** in the balloon cavity **177.**

In a deflation operation or step, the dilation controller **176** is operated to draw a flow of the inflation fluid **E** out of the balloon cavity **177** through the conduits **172, 170T.** The balloon **174** is thereby depressurized and deflated.

With reference to **FIGS. 1** and **3****,** the handle **128** and the faces **129A-F** provide an ergonomic shape that can be gripped by an operator (*e.g.,* physician) with one hand H like a handgun grip to position and navigate the probe **130** into, through and within the patient's anatomy. In use, the operator grasps the handle **128** such that the operator's palm **P** receives and bears against the rear face **129A** while one or more of the operator's fingers **F** are positioned opposite the palm **P** and bear against the front faces **129D-F.** Further, the operator may wrap his or her thumb **T** circumferentially around or alongside the handle **128,** or may place the thumb **T** over the shoulder **129C** and onto the top face **129B.** One or more of the operator's fingers **F** can grasp the upper front face **129D** while one or more of the operator's other (lower) fingers **F** grasp the lower front face **129E.** One or more of the operator's fingers **F** can operate the trigger **164B** as discussed below.

The ergonomic shape of the base **122** allows the handle to sit between the palm muscles and the opposing fingers providing an ergonomic solution to hold the handle by the operator. The device is further stabilized by positioning the thumb on to top of the handle. The ergonomic shape of the base **122** can thus provide the operator with improved dexterity, balance, and stability. The ergonomic shape of the base **122** enables the operator to effectively manipulate, position and operate the instrument **120** with a single hand holding the instrument **120.**

The ergonomic shape also enables the operator to conveniently control aspiration via the instrument **120** using the trigger, as discussed herein. The trigger **164B** is ergonomically located for displacement using the index and/or middle fingers.

In some embodiments, one or more control mechanisms (*e.g*., a button or switch) can be provided on the shoulder **129C** or top face **129B.** The design of the base **122** also enables the operator to conveniently and effectively access and actuate these mechanisms with the thumb of the single hand holding the base **122.**

Typically, the probe **120** will be inserted into the patient through a nostril and guided to the target region of the patient's anatomy with the balloon **174** in its deflated configuration. Typically, during these steps, the light source **151 is** activated in order to provide light for viewing the surgical field through the endoscope **20** and/or to provide trans-illumination through the patient's tissue. The operator can monitor the trans-illuminated light from external of the patient to identify the location of the probe tip **138A** relative to the known anatomical features of the patient.

The probe **120** may be bent as described above prior to insertion of the probe **120** into the patient.

The irrigation system **165** can be used to flow or spray irrigation fluid onto the endoscope **20** to clean the lens **22** of the endoscope. Advantageously, the instrument **120** can be used in this manner to clean the endoscope **20** without requiring removal of the endoscope or the instrument **120** from the patient.

The sinus treatment system **10** and the instrument **120** may be used, in accordance with methods of the invention, to execute surgical and treatment procedures (*e.g.*, balloon sinuplasty) using one or more of the operations or steps and functions described above. That is, for a given procedure, the operator can use the instrument **120** to aspirate, irrigate, deliver medication, dilate, and/or illuminate as described. Each of these operations or steps can be executed in a selected combination of operations or steps.

The aspiration, delivery, dilation, and/or illumination functions and operations can each be employed and executed independently of one another. Thus, for a given procedure, one or more of these operations can be omitted (*i.e.,* the functionality is not used). One or more of the operations can be executed simultaneously. One or more of the operations can be executed sequentially.

Each of the operations of aspiration, delivery, dilation, and illumination can be executed without removing the probe **130** from the patient between different functions and without replacing a component in or on the probe. For example, it is not necessary to remove and reconfigure the probe **130** between steps of irrigating and aspirating. However, it may be necessary or desirable to remove the probe **130** from the patient in order to reconfigure the instrument **120** between the irrigation fluid delivery and medication delivery operations.

The illumination function of the instrument **120** can be used in combination with any of the function combinations or methods described below.

The aspiration function and operation can be used to suction fluid and/or debris from the surgical field (*e.g.,* the paranasal sinuses). The fluid may include fluids that have been introduced to the surgical field by the operator (*e.g.*, irrigation fluid and/or medication) or fluids or debris originating in the patient (*e.g*., mucus or blood). In this way, the suction function can be used to remove undesirable material (*e.g*., infectious or blocking material) and clear the field for visualization. For example, during the procedure, the suction function and operation can be used to remove bleeding from resultant mucosal trauma or ostial dilation that might otherwise obscure the endoscopic view of surgeon and thereby hamper the procedure.

In some procedures, the system **10** and instrument **120** are used as follows to dilate a portion of the patient's anatomy using balloon sinuplasty. The balloon **174** is maintained in the non-expanded configuration or placed in the non-expanded configuration using the inflation controller **176.** With the balloon **174** deflated, the probe **130** is navigated into position in the patient anatomy (*e.g*., the paranasal sinuses). The balloon **174** is then inflated to the expanded configuration using the inflation controller **176** while in the selected position. The patient anatomy is thereby dilated and treated by the expansion of the balloon **174.** Thereafter, the balloon **174** is deflated to the non-expanded configuration using the inflation controller **176,** and removed from the patient. This dilation procedure may be supplemented with aspiration, delivery (irrigation fluid and/or medication), and illumination steps simultaneous with or in any selected sequence with the dilation steps.

In some procedures, the system **10** and instrument **120** are used to aspirate only, using the aspiration function.

In some procedures, the system **10** and instrument **120** are used to block or plug a passage of the patient anatomy with the expanded balloon **174,** and then aspirate a region ("the plugged region") partially or fully sealed by the plugging of the passage, using the aspiration function. In some procedures, the plugged passage is an ostia or frontal recess, and the plugged region is a sinus adjacent and in fluid communication with ostia or frontal recess.

In some procedures, the system **10** and instrument **120** are used to expand the balloon **174** in a region of the patient anatomy while simultaneously aspirating using the aspiration function.

In some procedures, the system **10** and instrument **120** are used to irrigate only, using the irrigation delivery function.

In some procedures, the system **10** and instrument **120** are used to block or plug a passage of the patient anatomy with the expanded balloon **174,** and then irrigate a region ("the plugged region") partially or fully sealed by the plugging of the passage, using the irrigation delivery function. In some procedures, the plugged passage is an ostia or frontal recess, and the plugged region is a sinus adjacent and in fluid communication with ostia or frontal recess.

In some procedures, the system **10** and instrument **120** are used to expand the balloon **174** in a region of the patient anatomy while simultaneously irrigating using the irrigation function.

In some procedures, the system **10** and instrument **120** are used to deliver medication only, using the medication delivery function.

In some procedures, the system **10** and instrument **120** are used to block or plug a passage of the patient anatomy with the expanded balloon **174,** and then deliver medication to a region ("the plugged region") partially or fully sealed by the plugging of the passage, using the medication delivery function. In some procedures, the plugged passage is an ostia or frontal recess, and the plugged region is a sinus adjacent and in fluid communication with ostia or frontal recess

In some procedures, the system **10** and instrument **120** are used to expand the balloon **174** in a region of the patient anatomy while simultaneously delivering medication using the irrigation function.

In some procedures, the system **10** and instrument **120** are used to irrigate, using the irrigation delivery function, and thereafter aspirate, using the aspiration function.

In some procedures, the system **10** and instrument **120** are used to block or plug a passage of the patient anatomy with the expanded balloon **174,** then irrigate a region ("the plugged region") partially or fully sealed by the plugging of the passage, using the irrigation delivery function, and then aspirate the plugged region, using the aspiration function. The aspiration may remove the irrigation fluid from the plugged region. In some procedures, the plugged passage is an ostia or frontal recess, and the plugged region is a sinus adjacent and in fluid communication with ostia or frontal recess.

In some procedures, the system **10** and instrument **120** are used to block or plug a passage of the patient anatomy with the expanded balloon **174,** then aspirate a region ("the plugged region") partially or fully sealed by the plugging of the passage, using the aspiration function, and then irrigate the plugged region, using the irrigation delivery function. The procedure may further include again aspirating the plugged region to remove the irrigation fluid, using the aspiration function. In some procedures, the plugged passage is an ostia or frontal recess, and the plugged region is a sinus adjacent and in fluid communication with ostia or frontal recess.

In some procedures, the system **10** and instrument **120** are used to aspirate, using the aspiration function, and irrigate, using the irrigation delivery function, simultaneously.

In some procedures, the system **10** and instrument **120** are used to plug a region of the patient anatomy with the expanded balloon **174,** then aspirate and irrigate the plugged region simultaneously.

In some procedures, the system **10** and instrument **120** are used to block or plug a passage of the patient anatomy with the expanded balloon **174,** then simultaneously aspirate and irrigate a region ("the plugged region") partially or fully sealed by the plugging of the passage, using the aspiration and irrigation functions. In some procedures, the plugged passage is an ostia or frontal recess, and the plugged region is a sinus adjacent and in fluid communication with ostia or frontal recess.

In some procedures, the system **10** and instrument **120** are used to deliver medication, using the medication delivery function, and thereafter aspirate, using the aspiration function.

In some procedures, the system **10** and instrument **120** are used to block or plug a passage of the patient anatomy with the expanded balloon **174,** then deliver medication to a region ("the plugged region") partially or fully sealed by the plugging of the passage, using the delivery function, and then aspirate the plugged region. The aspiration may remove an excess of the medication from the plugged region, using the aspiration function. In some procedures, the plugged passage is an ostia or frontal recess, and the plugged region is a sinus adjacent and in fluid communication with ostia or frontal recess.

In some procedures, the system **10** and instrument **120** are used to aspirate, using the aspiration function, and thereafter deliver medication, using the medication delivery function. The procedure may further include again aspirating the plugged region to remove excess medication, using the aspiration function.

In some procedures, the system **10** and instrument **120** are used to block or plug a passage of the patient anatomy with the expanded balloon **174,** then aspirate a region ("the plugged region") partially or fully sealed by the plugging of the passage, using the aspiration function, and then deliver medication to the plugged region, using the irrigation delivery function. The procedure may further include again aspirating the plugged region to remove excess medication, using the aspiration function. In some procedures, the plugged passage is an ostia or frontal recess, and the plugged region is a sinus adjacent and in fluid communication with ostia or frontal recess.

In some procedures, the system **10** and instrument **120** are used to aspirate, using the aspiration function, and deliver medication, using the medication delivery function, simultaneously.

In some procedures, the system **10** and instrument **120** are used to block or plug a passage of the patient anatomy with the expanded balloon **174,** then simultaneously aspirate and deliver medication to a region ("the plugged region") partially or fully sealed by the plugging of the passage, using the aspiration and delivery functions. In some procedures, the plugged passage is an ostia or frontal recess, and the plugged region is a sinus adjacent and in fluid communication with ostia or frontal recess.

Each of the foregoing procedures may be further modified to include expanding the balloon **174** simultaneously with one or more of the aspiration and fluid delivery steps. The step of expanding the balloon to plug or modify the patient anatomy can be executed simultaneously or independently of the other steps or functions executed using the instrument **120** (*i.e.,* aspiration, fluid delivery, and illumination).

Exemplary methods of use in accordance with some embodiments will now be described with reference to **FIGS. 12A-12F****.**

The system **10** and balloon dilation catheter instrument **120** may be particularly suited for treatment of the sinus outflow tract.

**FIG. 12A** illustrates a cross-sectional (coronal) view of a maxillary sinus and other associated anatomical structures of a subject (*i.e.,* a patient being treated).

**FIG. 12A** illustrates the cross-sectional (coronal) view of the maxillary sinus of the subject with the probe **130** of the instrument **120** (balloon dilation catheter) being advanced in the subject's nasal cavity toward a treatment target region.

**FIG. 12B** illustrates the cross-sectional (coronal) view of the maxillary sinus of the subject with the balloon **174** of the instrument **120** in a deflated or nonexpanded state and positioned in the maxillary ostia of the subject.

**FIG. 12C** illustrates the cross-sectional (coronal) view of the maxillary sinus of the subject with the balloon **174** of the instrument **120** being in an inflated or expanded state in the maxillary ostia of the subject. The balloon **174** has been inflated using the dilating system **170.**

**FIG. 12D** illustrates the cross-sectional (coronal) view of the maxillary sinus of the subject with the balloon **174** of the instrument **120** being in an inflated or expanded state in the maxillary ostia of the subject and the maxillary sinus being irrigated and suctioned at the same time using the aspiration system **160** and the delivery system **165.** By keeping the balloon **174** inflated during this process, there is very little excursion of fluid out of the maxillary ostia and into the nasal cavity and nasopharynx. This allows substantially all of the irrigated fluid to be suctioned back out of the maxillary sinus.

**FIG. 12E** illustrates the cross-sectional (coronal) view of the maxillary sinus of the subject with the balloon **174** of the instrument **120** being in an inflated or expanded state in the maxillary ostia of the subject and medications being applied to the maxillary sinus using the delivery system **165.** By keeping the balloon **174** inflated during this process there is little to no excursion of medications out of the maxillary sinus.

**FIG. 12F** illustrates cross-sectional (coronal) view of a dilated maxillary ostia of the subject after a balloon dilation procedure using the balloon **174.**

**FIG. 13A** illustrates the cross-sectional (sagittal) view of the frontal sinus and other associated anatomical structures of a subject (*i.e.,* a patient being treated).

**FIG. 13A** illustrates the cross-sectional (sagittal) view of the frontal sinus of the subject with the probe **130** of the instrument **120** being advanced in the subject's nasal cavity towards the frontal recess.

**FIG. 13B** illustrates the cross-sectional (sagittal) view of the frontal sinus of the subject with the balloon **174** of the instrument **120** in a deflated or nonexpanded state and positioned in the frontal recess of the subject.

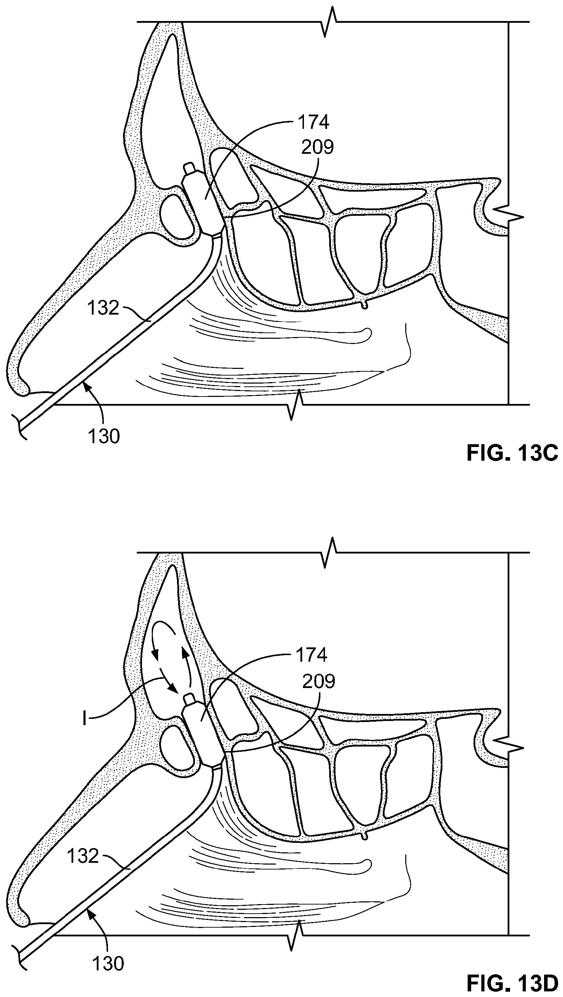

**FIG. 13C** illustrates the cross-sectional (sagittal) view of the frontal sinus of the subject with the balloon **174** of the instrument **120** being in an inflated or expanded state in the frontal recess of the subject.

**FIG. 13D** illustrates the cross-sectional (sagittal) view of the maxillary sinus of a subject with the balloon **174** of the instrument **120** being in an inflated or expanded state in the frontal recess of the subject and the frontal sinus being irrigated and suctioned at the same time using the aspiration system **160** and the delivery system **165.** By keeping the balloon inflated during this process there is very little excursion of fluid out of the frontal recess and into the nasal cavity and nasopharynx. This allows all the irrigated fluid to be suctioned back out of the frontal sinus.

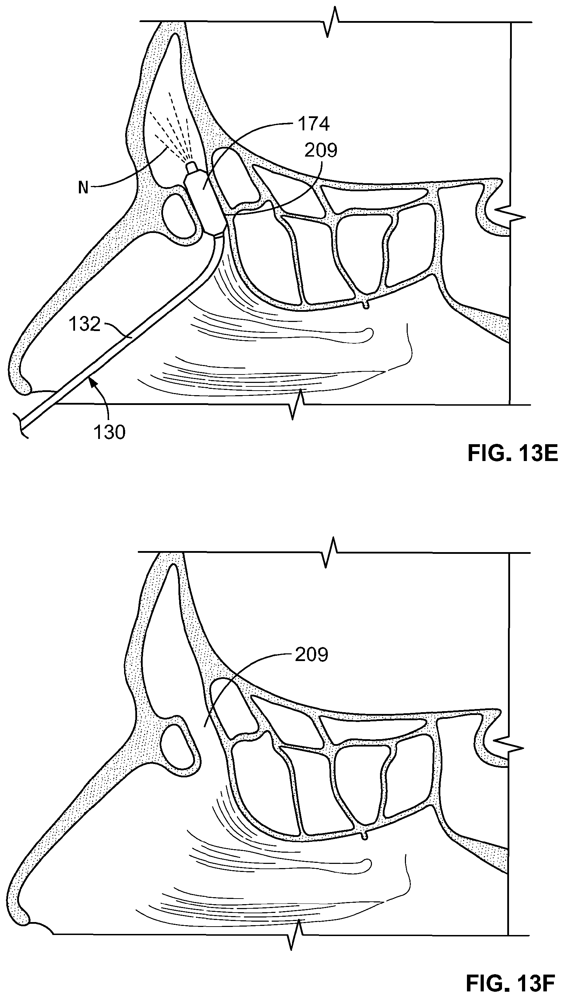

**FIG. 13E** illustrates the cross-sectional (sagittal) view of the frontal sinus of a subject with the balloon **174** of the instrument **120** being in an inflated or expanded state in the frontal recess of the subject and medications being applied to the frontal sinus using the delivery system **165.** By keeping the balloon inflated during this process there is little to no excursion of medications out of the frontal sinus.

**FIG. 13F** illustrates cross-sectional (sagittal) view of a dilated frontal recess after balloon dilation procedure using the balloon **174.**

During use, the probe **130** is manipulated and advanced across or into the anatomical space of interest. As shown in **FIGS. 12A, 12B****,** **13A** and **13B****,** the balloon **174** is typically in a deflated state during the time that the probe **130** is being moved or navigated into position and when the instrument **120** is being used for steps not requiring an expanded balloon **174.** After the probe **130 is** properly positioned in the sinus ostia or sinus outflow pathways, the balloon **174** is inflated.

During the procedure, any bleeding from resultant mucosal trauma or ostial dilation can obscure the endoscopic view of surgeon thereby hampering the procedure. The system **100,** instrument **120** and methods of the present invention provide a mechanism to suction out the mucus present in the sinus as well as a mechanism to irrigate the diseased sinus, and the capability to execute both activities simultaneously or independently, as desired.

Unlike known devices that are designed to be held like a pen between the operating surgeons thumb and forefinger with the device resting in the interdigital space between the thumb and forefinger, the instrument **120** employs a more ergonomic design. The ergonomic design of the instrument **120** places the dominant loads of the instrument in line with the weight bearing muscles of the operator's arm. This reduces undue weight pressure on the surgeon's forearm and wrist, which can reduce or prevent wrist cramps as compared to "pen" type devices. In addition, the ergonomic design of the instrument **120** extends the range of motion of the operator.

Using a probe **130** made up of rigid but malleable material enables the balloon dilation catheter **120** to be positioned without the need of a separate guiding catheter or guide wire in most, if not all, instances.

Having a suction or aspiration functionality permits the removal of blood and other secretions which makes it easier to visualize the placement of the balloon dilation catheter **120** during the procedure done using an endoscope.

The suction or aspiration system **160** may be used for suction or aspiration of blood or other secretions. The delivery system **165** may be used for delivery of fluids and/or medicaments to the sino-nasal cavity. By providing separate, coexisting conduits for suction and delivery, respectively, in the instrument **120** and the probe **130,** the instrument enables simultaneous suction and irrigation. This can prevent the flooding of the operating field by the irrigation fluid. In addition, thick secretions can be diluted by irrigating and made dilute enough to be suctioned/aspiration in the suction conduit. This keeps the endoscopic view of the operating field clear and allows for clear visualization of the anatomy.

The different shapes of the probe **130** may be factory-formed in a particular shape and offered as a different model as fully assembled. Alternatively, the probe **130** may be one of a set of replaceable, interchangeable or modular elements that can mounted inside the handle **128** using a slide and press-it type sealing arrangement. In yet another alternative, the shapes of the probe **130** could represent desirable shapes that a malleable inner guide member could be formed into by the user to better fit a particular application or subject's anatomy.

Light emitted from the distal end of the waveguide **154** at the distal end of the probe 130 can be used to guide for the proper placement of the probe **130.** In particular, the light can provide trans-illumination through the tissue of the patient, which the surgeon can observe and use to ascertain the location of the tip **138A** relative to the patient's anatomy. Also, the light emitted from the distal end of the waveguide **154** can help illuminate the surgical field for view through the endoscope **20.**

In other embodiments, the shaft **132** may include aspiration, delivery, and waveguide lumens of different cross-section shapes than those illustrated for the lumens **140, 142, 144.** For example, **FIG. 14** shows an alternative shaft **132'** including a waveguide lumen **140',** an aspiration lumen **142,** and an irrigation lumen **144.**

In other embodiments, the inflation conduit **172** may be replaced with a tubular sheath that surrounds the shaft **132** and forms an inflation channel in the space between the sheath and the outer diameter of the shaft **132.** In this case, the sheath may be substantially non-expandable or relatively non-expandable compared to the balloon **174.**

In further embodiments, the inflation conduit **172** and the balloon **174** may be replaced with a self-sealed balloon member as described below with regard to the balloon member **373.**

With reference to **FIGS. 15-21****,** a sinus treatment system **30** according to further examples is shown therein. The system **30** includes sinus treatment instrument system **300** and, optionally, an endoscope **20.** The system **30** and the instrument system **300** can be used in the same manner as the system **10** and the instrument system **100.** However, the instrument system **300** is differently constructed than the instrument system **100.**

The instrument subsystem **300** includes a handheld instrument **120,** the suction (negative pressure or vacuum) source **110,** the irrigation fluid source **112,** the medication source **114,** and the inflation fluid source **116.**

The instrument **320** is a balloon dilation catheter device that includes the same functional features and mechanisms in addition to the dilation functionality as described for the instrument **100.** The instrument **120** includes a handheld unit or base **322,** a probe (or guide member or extension assembly) **330,** a lighting system **350,** an aspiration system **360,** a delivery system **365,** and a dilation system **370** constructed and operative in the same manner as the base **122,** the probe **130,** the lighting system **150,** the aspiration system **160,** the delivery system **165,** and the dilation system **170,** except as discussed below. The instrument **320** has a proximal end **320A** and a distal end **320B** defining a primary or longitudinal axis **L-L.**

The probe **130** includes an elongate, tubular shaft **332** defining a longitudinal probe axis **P-P** extending from a proximal end **332A** to a distal end **332B.** The probe axis **P-P** is substantially parallel to the instrument axis **L-L.** The shaft **332** includes a dilator section **336,** an intermediate section **337,** and a tip section **338.** The tip section **338** terminates at a tip **338A.**

The shaft **332** has an outer surface **334A** and an inner surface **334B.** In some embodiments, the outer and inner surfaces **334A, 334B** are substantially cylindrical. The inner surface **334B** defines an axially extending shaft passage or lumen **333.** The shaft lumen **333** extends fully from a proximal end opening **333A** in the end **332A** to a distal end opening **333B** in the end **332B.**

The shaft **332** may be formed of any suitable material(s). In some embodiments, the shaft **332** is formed of a stainless steel hypotube.

In some embodiments, the shaft **332** is unitary. In some embodiments, the shaft **332** is unitarily formed. In some embodiments, the shaft **332** is monolithic. The shaft **332** may be molded (*e.g.,* injection molded) or extruded.

In some embodiments, the shaft **332** is formed of a rigid but malleable material. This permits the shaft **332** to be deliberately bent into a new shape or configuration in response to application of a sufficient bending force, and to retain the original or new shape or configuration when a lesser force is applied to the shaft **332.** In some embodiments, the required bending force is greater than any force the shaft **332** is expected or intended to experience in service during the surgical procedure (*i.e.,* during navigation or use of the probe **320** within the anatomy of the patient). The malleability of the shaft **332** may enable the user to bend the shaft **332** into a desired angle or curvature to achieve proper positioning for the balloon **374** and the distal end tip **338A** in the sinus ostia or sinus drainage pathways.

In some embodiments, the shaft **332** is pre-shaped to have a curved distal portion. The curved distal portion may be configured to match with the frontal sinus outflow tract or frontal recess.

In some embodiments, the distal portion the shaft **332** is formed right to the distal end **332B** and may have a radius of curvature in the range of from about 0.25 inch to about 1.5 inch and, in some embodiments, from about 0.75 to about 1.25 inch.

The shaft **332** extends a predetermined or prescribed distance or length **L3 (****FIG. 15****)** from the base **322** to the distal end **332B.** In some embodiments, the length **L3** is in the range of from about 3 to 10 inches and, in some embodiments, from about 5 to 7 inches.

In some embodiments, the probe **330** has an outer diameter in the range of from about 1 to 5 mm and, in some embodiments, from about 3 to 7 mm.

In some embodiments, the shaft **332** has a nominal wall thickness **T3 (****FIG. 19****)** in the range of from about 0.1 to 0.3 mm and, in some embodiments, from about 0.2 to 0.5 mm.

In some embodiments, the distal tip **338A** has an outer diameter in the range of from about 1 to 3 mm and, in some embodiments, from about 2 to 5 mm.

In some embodiments, the edges of the tip **338A** are rounded or smooth to prevent or reduce trauma to the mucosa of the sinuses.

The aspiration system **360** includes an aspiration conduit **362** in place of the conduit **162.** The conduit **362** extends through the handle **328.** A proximal end of the conduit **362** is fluidly connected to the suction port **360P.** The distal end of the conduit **362** is fluidly coupled to the proximal opening **333A** of the shaft lumen **333,** and thereby to an annular aspiration lumen **363** as discussed below. The conduit **362** further includes an integral, tubular tap or receiver leg **362A.** A receiver port **362B** is defined in the leg **362A** and is also fluidly connected to the proximal opening **333A.**

The aspiration conduit **362** may be formed of any suitable material. In some embodiments, the conduit **362** is formed of an elastomeric material. In some embodiments, the conduit **362** is formed of a flexible material. Suitable materials for the conduit **362** may include silicone, for example.

The delivery system **365** includes a conduit **366.** The conduit **366** defines a longitudinally extending delivery lumen **367** that terminates at a distal end opening **367B.**

The delivery conduit **366** extends from the delivery port **365P,** through the handle **328,** through the receiver port **362B,** through the distal portion of the conduit **362,** through the shaft lumen **333,** and the distal end **332B.** In some embodiments, the terminal end and distal end opening **367B** of the delivery conduit **366** are located substantially at the distal end opening **333B.**

In some embodiments, a fluid-tight seal is provided between the conduit **366** and the conduit **362** at the port **362B.**

The delivery conduit **366** may be formed of any suitable material. In some embodiments, the conduit **366** is formed of an elastomeric material. In some embodiments, the conduit **366** is formed of a flexible material. Suitable materials for the conduit **366** may include silicone, for example.

The dilation system **370** includes a balloon member **373** in place of the conduit **172** and the balloon **174.** The balloon member **373** is a self-sealed balloon unit. The balloon member **373** includes a conduit **372,** an integral dilation section **374,** and an integral nondilation section **375.**

The dilation section **374** is a balloon. The balloon **174** is donut-shaped and includes a tubular inner wall **374A** and a tubular outer wall **374B** defining an enclosed, annular balloon chamber **374C** radially therebetween.

The nondilation section **375** includes a tubular inner wall **375A** and a tubular outer wall **375B** defining an enclosed, annular inflation passage **375C** radially therebetween. The passage **375C** fluidly connects the conduit **372** to the balloon chamber **374C.**

The balloon **374** is mounted proximate the distal end **332B.** The balloon member **373** may be bonded to the shaft **332** using a weld, adhesive, or the like. Alternatively, the balloon **374** may be secured to the shaft **332** using a mechanical connection.

The dilation system **370** is configured to selectively inflate and deflate the balloon **374** on demand by the operator as described above for the balloon **174.** The balloon **374** is pliable and expandable when inflated. The dilation system **170** can be operated to place the balloon **374** alternatively in at least a first, relatively radially non-expanded configuration (herein, the non-expanded position) and a second, radially expanded configuration (herein, the expanded configuration). In the expanded configuration, the outer diameter **D4 (****FIG. 18****)** of the balloon **374** is larger than in the deflated configuration.

The nondilation section **375** is non-expandable or less expandable than the balloon **374** so that, when the balloon **374** is inflated and expanded, the nondilation section **375** will not radially expand or will expand to a substantially lesser extent than the balloon **374.**

In some embodiments and as will be appreciated from the description herein, the dilation system 370 can be operated to place the balloon **374** in a range of different expanded configurations. That is, the balloon **374** can be forced to assume one of a plurality of different expanded configurations each having a different outer diameter **D4.**

In some embodiments and as will be appreciated from the description herein, the non-expanded configuration of the balloon **374** may not be a fully non-expanded or deflated configuration. That is, the outer diameter of the balloon **374** may be greater than is smallest possible diameter in the non-expanded configuration.

In some embodiments, the main section **374A** takes on a cylindrical frusto-conical shape when the balloon **374** is substantially fully inflated.

In some embodiments, the balloon **374** has dimensions as described above for the balloon **174.**

The balloon **374** may be formed of any suitable material. In some embodiments, the balloon **374** is formed of a material and/or using a technique as described above for the balloon **174.**

As shown in **FIGS. 18** and **19****,** a longitudinally extending space **353** is defined between the outer surface **334A** of the shaft **332** and the inner wall **374A** of the balloon member **373.**

The integral lighting system **350** includes a waveguide **354** constructed and operative in the same manner as the waveguide **154.** In some embodiments, the waveguide **354** is an optical fiber (*e.g.,* a polymeric or glass optical fiber) and may include a sheath to protect the waveguide and prevent light loss. The waveguide **354** extends from the light source **351** and longitudinally through the space **353** to a waveguide distal end **354B** adjacent the shaft distal end **332B.** The waveguide **354** has an end face **354C** at the distal end **354B,** from which light is emitted. In some embodiments, the waveguide distal end **354B** is located substantially flush with the distal end **332B.** In other embodiments, the waveguide distal end **354B** extends outwardly beyond the distal end **332B** a distance in the range of from about 1 to 5 cm.

It can be seen in **FIG. 19** that the shaft **332** and the delivery conduit **366** nested therein define generally concentric aspiration and delivery lumens. In particular, the delivery conduit **366** defines an inner or generally central delivery lumen **367.** The shaft **332** and the delivery conduit **366** define the annular aspiration lumen radially surrounding but fluidly partitioned or separated from the delivery lumen **367.**

In some embodiments and as shown in **FIG. 19****,** the lumens **363** and **367** may have different cross-sectional areas from one another. In some embodiments, the cross-sectional area of the aspiration lumen **363** is greater than the cross-sectional area of the delivery lumen **367.** In some embodiments, the cross-sectional area of the aspiration lumen **363** is at least 1.5 times the cross-sectional area of the delivery lumen **367.** In some embodiments, the cross-sectional area of the aspiration lumen **363** is in the range of from about 2 to 4 times the cross-sectional area of the delivery lumen **367.**

In some embodiments, the cross-sectional area of the aspiration lumen **363** is in the range of from about 0.28 to 1.75 mm².

In some embodiments, the cross-sectional area of the delivery lumen **367** is in the range of from about 0.07 to 0.8 mm².

In alternative embodiments, the instrument **320** is modified so that the inner lumen **367** is used to aspirate and the outer lumen **363** is used to deliver fluids (*e.g.*, irrigation fluid and/or medication).

Balloon inflation devices and methods according to embodiments of the invention can be used for the treatment of diseased paranasal sinuses. More particularly, embodiments can provide minimally invasive, balloon based systems and methods for dilating the sinus ostium or drainage pathways of human paranasal sinuses in the treatment of chronic rhinosinusitis and other related disorders.

In some embodiments, a balloon dilation device includes a handle shaped like a gun holder, a rigid shaft that is coupled to an expandable balloon, a rigid shaft with tubing system to simultaneously suction, irrigate, and/or deliver medication to surgically dilate the anatomically stenotic segments of the paranasal sinuses (namely: maxillary, frontal and sphenoid sinus) by placing the balloon in the ostia and drainage pathways of the sinuses. The balloon is inflated to expand or remodel the drainage pathway.

In some embodiments, a device is provided for balloon dilation of anatomically stenotic segments of the paranasal sinuses in humans, for delivering medications to the paranasal sinuses and for collecting fluid or tissue specimens from the paranasal sinuses for diagnostic purposes. A device for dilating an ostium of a paranasal sinuses may include: a handle in a shape that utilizes the palm of the hand and apposition fingers to stabilize the device; an elongate shaft that is easily moldable having a proximal end coupled with the handle and extending to a distal end; a dilator balloon mounted on the shaft and having a non-expanded configuration and an expanded configuration.

In some embodiments, a device includes an ergonomically designed handle piece, a rigid probe coupled to an inflatable balloon on an outside surface of the probe, and a system of tubes on the inside of the probe to carry fiber optic from a light source housed in the handle piece, and a system of tubing and valves that allow for simultaneous suction and irrigation independent of the inflation status of the balloon. The handle piece may be configured to be held by the operating surgeon in his palm whereby the handle sits between the muscles of the palm and is opposed by the four fingers with the index finger controlling a suction trigger. The handle piece is further stabilized by the thumb from the top. This results in distribution of the weight of the system in line with the axis of the muscles of the arm. The rigid probe can be bent at various angles to navigate the sino-nasal passageways and helps in positioning the balloon at the correct location. Curvature and longitudinal location of the curved segment is configured by the operating surgeon.

In some embodiments, the balloon **174** is coated with one or more medicaments at one or more predetermined locations along the length of the balloon **174.** This enables the delivery of medicaments to the area or the sinuses dilated by the balloon **174.**

Because the sizes of the sinus openings and the depths of the sinuses are different for maxillary/frontal/sphenoid sinuses resulting in different portions of the balloon **174** being in contact with maxillary, frontal and sphenoid sinus openings, respectively, the balloon **174** may be coated with medicaments at three different locations on the balloon **174,** and these medicaments can be transferred to the respective sinus openings.

It will be appreciated that the system **10** may include items such as a vacuum pump, reservoirs for dispensing and collecting fluids, a power supply, pumps, hydraulic cylinders, pressure sensors, flow control valves, and/or electronic controls as parts of or supplemental to the suction source **110,** the irrigation fluid source **112,** the medication source **114,** the aspiration system **160,** the delivery system **165,** and the dilation system **170.**

In some embodiments, the Luer lock connectors of the ports **160P, 165P, 170P** are recessed into the profile of the handle **128.**

According to further embodiments, the dilation system may include a trigger or other control mechanism integral with and on the handle **128** and that is can be selectively operated to inflate and deflate the balloon **174, 374.** In some embodiments, an inflation mechanism (*e.g*., a pump or syringe) and/or an inflation fluid reservoir are integrated into the handle **128.**

According to further embodiments, the delivery system may include a trigger or other control mechanism integral with and on the handle **128** and that is can be selectively operated to control delivery of fluid (*e.g*., irrigation fluid and/or medication) through the delivery lumen **144, 367.** In some embodiments, an transfer mechanism (*e.g*., a pump or syringe) and/or a fluid reservoir (*e.g*., containing irrigation fluid and/or medication) are integrated into the handle **128.**

In some embodiments, the instruments **120, 320** may include one or more interchangeable or replaceable modular components. For example, the instrument **120** can be configured such that the probe **130** is releasably coupled to the base **122.** After the instrument **120** has been used to conduct a procedure, the probe **130** can be removed and discarded, recycled, or cleaned and sterilized, for example. A new probe **130** can then be mounted on and coupled to the base **122** and the base **122** can be re-used with the new probe **130** to conduct another procedure. Thus, an instrument according to some embodiments (*e.g.,* the instrument **120** or the instrument **320)** may include a combination of re-usable and disposable components.

According to some embodiments, the balloon **174, 374** of each instrument **120, 320** is substantially non-compliant. That is, the ability of the balloon material to stretch beyond a prescribed expansion size as the pressure is increased and thereby further expand the size of the balloon is very small. The balloon may be pleated and wrapped onto the shaft **132, 332** in its un-inflated position to provide a small initial uninflated balloon profile. When the balloon is inflated, it will unwrap to an initial fully inflated balloon diameter. Then, over quite a small range of plastic deformation, the balloon can be further expanded with additional pressure (via the pressurized fluid). The nominal diameter (*i.e.,* the nominal inflated balloon diameter measured at a specified pressure) of balloon may be set at a pressure between the pressure required to fully expand the pleats and unwrap the balloon and the pressure that would cause the balloon to burst ("rated burst pressure"). Relatively high balloon inflation pressures as described herein may be required to provide sufficient force against the sinus anatomy and, in particular, to push back tissues and break small bones in the sinuses.

In some embodiments, the balloon **174, 374** is inflated multiple times during a procedure on a patient to execute multiple dilation procedures as described herein. These multiple balloon dilations can be applied to the same anatomy or to different locations in the sinuses.

In some embodiments, the instrument **120, 320** is configured and used to deliver a medication (*e.g*., a drug formulation in solution, suspension or emulsion) through the balloon **174, 374.** In this case, the medication may be included in the inflation fluid. When the balloon is pressurized and inflated, a portion of the inflation fluid will seep or weep out of the balloon (*e.g*., through small pores) and onto the surrounding anatomy. In this case, a portion of the balloon wall may be formed of a microporous membrane through which the medication passes.

With reference to **FIGS. 22-31****,** a sinus treatment instrument system **400** according to embodiments of the invention is shown therein. The sinus treatment instrument system **400** may be used in place of the sinus treatment instrument system **300** in the sinus treatment system **10 (****FIG. 1****),** and may be used along with an endoscope **20** as described herein. The instrument system **400** can be used in the same manner as the system **10** and the instrument system **100.** However, the instrument system **400** is differently constructed than the instrument system **100.**

The instrument subsystem **400** includes a handheld instrument **420,** the suction (negative pressure or vacuum) source **110,** the irrigation fluid source **112,** the medication source **114,** and the inflation fluid source **116.**

The instrument **420** is a balloon dilation catheter device that includes the same functional features and mechanisms in addition to the dilation functionality as described for the instrument **100.** The instrument **420** includes a handheld unit or base **422,** a probe (or guide member or extension assembly) **430,** a lighting system **450,** an aspiration system **460,** a delivery system **465,** and a dilation system **470** constructed and operative in the same manner as the base **122,** the probe **130,** the lighting system **150,** the aspiration system **160,** the delivery system **165,** and the dilation system **170,** except as discussed below. The instrument **420** has a proximal end **420A** and a distal end **420B** defining a primary or longitudinal axis **L-L.** The base **422** includes a handle **428** corresponding to the handle **128.**

With reference to **FIGS. 23** and **24****,** the probe **430** includes an elongate, tubular outer shaft **432,** an elongate, tubular inner shaft **480,** an inner cover sleeve **484,** a transition cover sleeve **485,** a distal cover sleeve **486,** and an atraumatic tip **488.** The probe **430** defines a longitudinal probe axis **P-P** substantially parallel to the instrument axis **L-L.** The probe **430** includes a dilator section **436,** an intermediate section **437,** and a tip section **438.** The tip section **438** includes the atraumatic tip **488** and terminates at a distal end **430B** of the probe **430.**

The inner shaft **480** extends from a proximal end **480A** to a distal end **480B**. A shaft lumen **481** extends fully from a proximal end opening **481A** to a distal end opening **481B** at the probe distal end **432B.** The inner shaft **480** includes a distal extension section **480E,** which includes the dilator section **436.**

The inner cover sleeve **484** surrounds the inner shaft **480** from a location proximate the proximal end **480A** to the proximal end of the dilator section **436.** An elongate waveguide **454** (*e.g.,* an optical fiber) corresponding to the waveguide **154** extends axially through the inner cover sleeve **484** radially between the inner shaft **480** and the inner cover sleeve **484.** In some embodiments, the inner cover sleeve **484** is translucent. In some embodiments, the inner cover sleeve **484** is formed of a polymeric material. In some embodiments, the inner cover sleeve **484** is formed of a heat shrink material, and is heat shrunk onto the inner shaft **480** and the waveguide **454** to firmly secure the waveguide **454** in place on the inner shaft **480.**

The dilation system **470** includes a dilation balloon member **473** constructed and operable in the same manner as described for the balloon member **373.** The balloon member **473** is a self-sealed balloon unit. The balloon member **473** includes a conduit **472,** an integral dilation section or balloon **474,** and an integral, tubular nondilation section **475.** The nondilation section **475** surrounds the inner cover sleeve **484.**

The dilation section **474** is a balloon. The balloon **474** is single-wall balloon. However, the balloon **474** may be otherwise constructed (*e.g.*, a donut-shaped, double-walled structure as described for the balloon **374).** The balloon **474** is mounted on dilator section **436** proximate the probe distal end **430B.** The balloon member **473** may be bonded to the inner cover sleeve **484** using adhesive or the like. Alternatively, the balloon **474** may be secured to the shaft **332** using a mechanical connection. In some embodiments, at least the side wall **474A** of the balloon **474** is translucent.

The conduit **472** is connected or connectable to the inflation fluid source **116** via a connector or port **470P** in the handle **422** to selectively inflate the balloon as described herein with regard to the balloon **174.**

The outer shaft **432** extends from a proximal end **432A** proximate the base **422** to a distal end **432B** at the proximal end of the distal extension section **480E** of the inner shaft **480.** The outer shaft **432** defines an axially extending shaft passage or lumen **433.** The shaft lumen **433** extends fully from end **432A** to end **432B** and terminates at a respective opening at each end **432A, 432B.** The subassembly of the inner shaft **480,** the inner cover sleeve **484,** the waveguide **454,** and the nondilation section **475** extends axially through and distally beyond the lumen **433.**

The distal extension section **480E** of the inner shaft **480** extends distally from the outer shaft distal end **432B** to the inner shaft distal end **480B.** In some embodiments, the distal extension section **480E** has a length **L6 (****FIG. 25****)** in the range of from about 20 mm to 40 mm.

The distal cover sleeve **486** surrounds a portion of the outer shaft **432** and extends distally beyond the outer shaft end **432B** to cover the distal edge of the outer shaft **432.** The transition cover sleeve **485** covers the transition from the base **422** to the probe **430.** In some embodiments, the cover sleeves **485, 486** are formed of a polymeric material. In some embodiments, the cover sleeves **485, 486** are formed of a heat shrink material, and are heat shrunk about the outer shaft **432.**

The atraumatic tip member **488** is affixed to the distal end **480B** of the inner shaft **480.** For example, the atraumatic tip member **488** may be bonded to the distal end **480B.** The atraumatic tip member **488** surrounds the distal end portion of the waveguide **454.** In some embodiments, the atraumatic tip member **488** projects forwardly (distally) beyond the distal end **480B** of the inner shaft **480** and the distal end **454B** of the waveguide **454** a distance **L8 (****FIG. 25****).** In some embodiments, the distance **L8** is in the range of from about 0.1 mm to 2 mm.

The atraumatic tip member **488** is formed of a softer material than the inner shaft 480. In some embodiments, the atraumatic tip member **488** is formed of a polymeric material. In some embodiments, the atraumatic tip member **488** is formed of an elastomer. In some embodiments, the atraumatic tip member **488** is formed of a thermoplastic elastomer. In some embodiments, the atraumatic tip member 488 is formed of a polyether block amide (e.g., PEBAX elastomer). In some embodiments, the atraumatic tip member **488** is formed of an elastomer having a hardness in the range of from about Shore D25 to Shore D 50. In some embodiments, the atraumatic tip member **488** is translucent.

The atraumatic tip member **488** can serve to prevent or reduce trauma to the mucosa of the sinuses. In particular, the atraumatic tip **488** can prevent scratching of the sinus and nasal cavity tissues as the balloon **474** enters the nasal cavity and thereby prevent or reduce bleeding. Bleeding may hinder the visualization during surgery and impair proper placement of the balloon **474.**

The inner shaft **480** may be formed of any suitable material(s). In some embodiments, the inner shaft **480** is formed of a stainless steel hypotube.

In some embodiments, the inner shaft **480** is formed of a rigid but malleable material. This permits the inner shaft **480** to be deliberately bent into a new shape or configuration in response to application of a sufficient bending force, and to retain the original or new shape or configuration when a lesser force is applied to the inner shaft **480.** In some embodiments, the required bending force is greater than any force the inner shaft **480** is expected or intended to experience in service during the surgical procedure (*i.e.,* during navigation or use of the probe **420** within the anatomy of the patient). The malleability of the inner shaft **480** may enable the user to bend the distal extension section **480E** of the inner shaft **480** into a desired angle or curvature to achieve proper positioning for the balloon **474** and the distal end tip **438** in the sinus ostia or sinus drainage pathways.

The outer shaft **432** may be formed of any suitable material(s). In some embodiments, the outer shaft **432** is formed of a stainless steel hypotube.

In some embodiments, the outer shaft **432** is more rigid than at least the distal extension section **480E** of the inner shaft **480.** In some embodiments, the outer shaft **432** is fully rigid so that it will not bend substantially or at all in use. However, in other embodiments, the outer shaft **432** and the inner shaft **480** may both be malleable, with the inner shaft **480** being more easily bent.

A fluid connector fitting or manifold **478 (****FIGS. 23** and **28****)** is seated in the base **422.** The connector manifold **478** includes an aspiration channel **478A** and a delivery channel **478B** that converge into a shared channel **478C.** The distal end of the connector manifold **478** is mated and secured to the proximal end **480A** of the inner shaft **480** such that the distal opening of the shared channel **478C** is fluidly connected to the shared lumen **481.** The connector manifold **478** may be formed of any suitable material (*e.g.*, elastomer) and may be secured to and sealed with the proximal end of the inner shaft **480** by any suitable technique (*e.g*., interference fit or adhesive).

The aspiration system **460** includes an aspiration conduit **462** and an aspiration controller **464.** The aspiration conduit **462** extends through the handle **428.** A proximal end of the conduit **462** is fluidly connected to a suction port **460P,** which is in turn connected to the suction source **110.** The distal end of the conduit **462** is fluidly coupled to the proximal opening of the inner shaft lumen **481** by the connector manifold **478.**

The delivery system **465** includes a delivery conduit **466** extending through the handle **428.** A proximal end of the conduit **466** is fluidly connected to a delivery port **465P,** which is in turn connected to the irrigation fluid source **112** or the medication source **114.** The distal end of the conduit **466** is also fluidly coupled to the proximal opening of the inner shaft lumen **481** by the connector manifold **478.**

The aspiration controller **464** includes a valve **464A** in the base **422,** an integral trigger **464B** attached to the base **422,** a return spring **464C,** and a bearing feature or cross bar **464D.** The trigger **464B** is operable to selective allow and prevent fluid flow between the lumen **481** and the suction source **110** through the conduit **462.**

The valve **464A** operates as a pinch valve. A section of the conduit **462** extends through a hole **464E** in the trigger **464B.** When the trigger **464B** is not depressed, the spring **464C** forces the trigger **464B** in a forward direction **TF** so that the trigger **164B** compresses the conduit **462** against the cross bar **464D** and closes the conduit **462.** When the trigger **464B** is depressed against the force of the spring **464C,** the conduit **462** is opened. Upon release, the trigger **464B** is urged back to the closed position by the spring **464C.** The trigger **164B** thereby operates as a hand-actuated valve **464A** that can be selectively operated by the user to open and close the fluid pathway between the suction source **110** and the lumen **481.**

In alternative embodiments, the trigger **464B** is configured such that its operation is reversed. In that case, the conduit **462** is open by default. When the trigger **464B** is depressed, the conduit **462** is closed. Upon release, the trigger **464B** is urged back to the open position by the spring **464C.**

With reference to **FIGS. 23** and **29****-31,** the integral lighting system **450** includes a light source **451** such as an LED, a battery **452,** a waveguide **454,** and a heatsink member **453.** The lighting system **450** may be constructed and operated in the same manner as described for the lighting system **150,** except as discussed below.

The waveguide **454** constructed and operative in the same manner as the waveguide **154.** In some embodiments, the waveguide **454** is an optical fiber (*e.g.,* a polymeric or glass optical fiber) and may include a sheath to protect the waveguide and prevent light loss.

In some embodiments, the light source **451** is as an LED. In some embodiments, the LED **451** is mounted on a PCB **451A.** The LED **451** is connected to and powered by the battery **452.**

The waveguide **454** extends from a proximal end **454A** at or adjacent the light source **451** to a waveguide distal end **454B** adjacent the probe distal end **430B.** The waveguide **454** has an end face **454C** at the distal end **454B,** from which light is emitted. In some embodiments, the waveguide distal end **454B** is located substantially axially flush with the probe distal end **430B** (*e.g.,* the distal end of the atraumatic tip **488).**

The heatsink member **453** includes a through hole **453A** and a recess **453B** surrounding the through hole **453A** on a rear side **453D** of the heatsink member **453.** The LED **451** faces the rear side **453D** and located in or closely adjacent the recess **453B.** The waveguide **454** extends from proximate the LED **451** and through the through hole **453A.** In some embodiments, the heatsink member **453** is formed of metal.

In use, the heatsink member **453** serves to absorb and dissipate heat generated by the LED **451** so that the heat does not damage or unduly affect the performance of surrounding components. The heatsink member 453 is also designed to reduce scatter of light from the LED **451** and to transmit the majority of the light into the waveguide **454,** so that there is minimal loss. The heatsink member **453** can enable placement of the waveguide end close to the LED **451.**

With reference to **FIG. 25****,** in the invention, the balloon **474** is illuminated by the waveguide **454** when the LED **451** is operated. In the illustrated embodiment, a distal end section **454E** of the waveguide **454** extends distally beyond the distal end of the distal cover sleeve **486** to the distal end of the atraumatic tip **488.** The waveguide **454** is constructed such that a portion **EA** of the light from the LED **451** is emitted (substantially in a forward direction) from the waveguide **454** through the waveguide end face **454C,** and another portion **ER** of the light from the LED **451** is emitted (substantially in radially outward directions) from the sidewall surface of the waveguide **454** in the distal end section **454E.** The radial light **ER** is transmitted through the translucent inner cover sleeve **484,** the translucent side wall **474A** of the balloon **474,** and the translucent atraumatic tip **488.** In some embodiments, any light emitted from the waveguide **454** inboard of the distal end section **454E** is blocked by the base **422,** the outer shaft **432,** or the cover sleeve **486** so that only the light emitted from the end face **454C** and the distal end section **454E** is visible externally of the instrument **420** along the probe **430.**

The emitted and transmitted radial light **ER** presents an elongate, axially extending, visible line or band **LB** of light along the length of the balloon **474.** In use, this illumination of the balloon **474** transluminates through the patient tissue, where is it visible to the physician. The physician can use this translumination to better determine the placement or position of the entire balloon **474** in the patient's sinus region.

In some embodiments, the light band **LB** spans at least the full length of the balloon **474.** In some embodiments, the length **L7 (****FIG. 25****)** of the light band **LB** is in the range of from about 20 mm to 40 mm. The radial light **ER** may be emitted in multiple directions about the circumference of the probe **420.** For example, the light band **LB** may be visible about 360 degrees of the probe **420** or less than 360 degrees.

A visible balloon position marker **479** may be located at an intermediate axial location along the length of the balloon **474.** In some embodiments, the balloon position marker **479** is located at the axial midpoint of the balloon **474.** The balloon marker **479** may be a band of ink or elastomer that visibly contrasts with the inner cover sleeve **484,** for example.

In use, the physician may view (using the endoscope **20)** and reference the balloon marker **479** to better assess the position of the balloon **474** in the patient's sinus cavity during placement of the balloon **474** prior to inflation of the balloon **474.** The marker **479** can be used in this manner as a depth guide to indicate to the surgeon how deep the balloon **474** is in the sinus. It may be important for the surgeon to know where the distal end of the balloon **474** is when the distal end of the balloon **474** is not visible. Generally, the distal end of the balloon **474** will not be visible when the balloon **474** has entered the sinus cavity, when the surgeon can only see the proximal part of the balloon **474** using the endoscope **20.**

The instrument **420** may be modified to include aspects or features as described elsewhere herein in accordance with other embodiments. For example, whereas the illustrated instrument **420** includes a single lumen **481** that serves as both an aspiration lumen and a fluid delivery lumen, in other embodiments, the instrument may include separate dedicated aspiration and delivery lumens as described with reference to the instruments **120, 320,** for example.

It will be appreciated that while treatment systems, instrument systems, and instruments have been described herein as used to treat a sinus of a subject (*e.g.,* a paranasal sinus), instrument systems (*e.g*., **100, 300, or 400)** and instrument systems (e.g., **120, 320,** or **420)** according to embodiments of the invention may be used to treat anatomy of a patient (including other passages or cavities) other than sinuses.

In accordance with some methods of the invention and with reference to **FIG. 32****,** the sinus treatment instrument system **400** is used to dilate a eustachian tube of the subject. In accordance with methods of the invention, the probe **430** is inserted trans-nasally through the nasopharynx. The balloon **474** is positioned in the lower portion of the eustachian tube, and then inflated at that location to dilate the eustachian tube. The operator continues to inflate the balloon **474** as needed to sufficiently dilate the eustachian tube. The operator then deflates the balloon **474** and removes the probe **430** from the eustachian tube and the patient.

During this process, the balloon **474** effectively airtight plugs or seals the eustachian tube at its inner or lower end, thereby sealing the tympanic cavity. Ordinarily, the volumetric displacement caused by the balloon as it further expands in the eustachian tube (after sealing the tympanic cavity) would cause the air pressure in the tympanic cavity to increase.

In accordance with methods of the invention, the operator also operates the aspiration system **460** (including the suction source **110)** to apply suction to aspirate or draw material out of the eustachian tube and/or the tympanic cavity through the distal end port **481B** while the dilation balloon **474** is disposed in the eustachian tube, is at least partly expanded (*e.g*., inflated), and is plugging the eustachian tube such that the dilation balloon **474** substantially prevents the escape of fluid from the tympanic cavity or inner eustachian tube past the dilation balloon **474.** In some embodiments, the material aspirated out of the eustachian tube and/or the tympanic cavity includes fluid (liquid and/or gas). The aspirated fluid may include air. The excess pressure that may ordinarily be developed in the tympanic cavity during the balloon expansion or inflation process is offset or relieved (reduced or eliminated) by this suction and depressurization. In this way, the aspiration serves to prevent damage to the middle ear and inner ear structures from excessive increase in pressure during the eustachian tube dilation process.

In accordance with some methods as described above, the operator operates the aspiration system **460** to apply the suction (to aspirate or draw material out of the eustachian tube and/or the tympanic cavity through the distal end port **481B)** simultaneously with expanding the dilation balloon **474.** For example, in some embodiments the aspiration system **460** draws material out of the eustachian tube and/or the tympanic cavity at the same time that the balloon inflation source is adding inflation fluid into the inner chamber of the dilation balloon **474** to expand the dilation balloon **474.**

In accordance with some methods as described above, the operator operates the aspiration system **460** (to apply the suction to aspirate or draw material out of the eustachian tube and/or the tympanic cavity through the distal end port **481B)** at a time when the dilation balloon **474** is not expanding but after at least partially expanding the dilation balloon. In some embodiments, the aspiration system **460** draws material out of the eustachian tube and/or the tympanic cavity at a time or times when the dilation balloon is expanded and forms an airtight seal in the eustachian tube, but the balloon is not currently being expanded or inflated. In some embodiments, the instrument system **400** is operated to inflate the balloon **474** and aspirate from the eustachian tube and/or the tympanic cavity in multiple alternating steps (*e.g*., partially inflate the balloon, then aspirate, then further inflate the balloon, and then aspirate again).

The atraumatic tip **488** can facilitate entry into the eustachian tube through the trans-nasal approach without causing undue trauma to the surrounding tissues.

The flexible shaft **484** allows for the distal end portion of the probe **430** to be bent or molded to fit the anatomy of the trans-nasal approach (e.g., according to the patient's personally anatomy). In particular, the portion of the flexible shaft **484** covered by the balloon **474** can be bent or molded to fit the anatomy of the trans-nasal approach.

Many alterations and modifications may be made by those having ordinary skill in the art. Therefore, it must be understood that the illustrated embodiments have been set forth only for the purposes of example, and that it should not be taken as limiting the invention as defined by the following claims. The following claims, therefore, are to be read to include not only the combination of elements which are literally set forth but all equivalent elements for performing substantially the same function in substantially the same way to obtain substantially the same result. The claims are thus to be understood to include what is specifically illustrated and described above, what is conceptually equivalent, and also what incorporates the essential idea of the invention.

## Claims

1. An instrument system (400) for treating a subject, the instrument system comprising:
an instrument (420) including:
a base (422) configured to be gripped by an operator;
an elongate probe (430) including a probe proximal end (480A) coupled with the base and extending to a probe distal end (480B), the probe including at least one lumen (481) terminating at at least one port (481B) proximate the probe distal end; and
a dilation balloon (474) mounted on the probe proximate the probe distal end, wherein the dilation balloon is expandable into an expanded configuration to execute a dilation operation; and
an integral lighting system (450) including:
a light source (451); and
a waveguide (454) on the probe and having a light emitting end (454B) proximate the probe distal end, wherein the integral lighting system is configured to transmit light through the waveguide from the
light source to the light emitting end;
wherein:
a section (454E) of the waveguide extends through the dilation balloon; and wherein the integral lighting system is so configured that
when the light source is operated, light (ER) from the light source is transmitted through the waveguide, emitted radially outwardly through a sidewall of the waveguide, and transmitted through a side wall of the dilation balloon.

2. The instrument system of Claim 1 wherein the integral lighting system includes a battery (452) to power the light source, and optionally,
wherein the light source and the battery are mounted in the base.

3. The instrument system of Claim 1 wherein the light source (451) includes a light emitting diode (LED).

4. The instrument system of Claim 1 further including an endoscope (20).

5. The instrument system of Claim 1 wherein the instrument includes a heat sink member (453) located adjacent the light source.

6. The instrument system of Claim 5 wherein:
the heat sink member (453) includes a through hole (453A); and
the waveguide extends from the light source through the through hole to the light emitting end.

7. The instrument system of Claim 5 wherein the heat sink member (453) is formed of metal.

8. The instrument system of Claim 1 wherein:
the section (454E) of the waveguide is configured to emit an axially extending band of the light through the sidewall of the waveguide; and
the band of light substantially spans a full axial length of the dilation balloon (474).

9. The instrument system of Claim 1 wherein:
the probe includes:
an elongate, tubular outer shaft (432), wherein the outer shaft is rigid; and
an elongate, tubular inner shaft (480) extending through the outer shaft,
wherein the inner shaft is malleable;
the inner shaft includes a distal extension section (480E) extending distally beyond the outer shaft toward the probe distal end;
the at least one lumen extends through the inner shaft; and
the dilation balloon (474) is mounted on the distal extension section.

10. The instrument system of Claim 1 wherein:
the at least one lumen includes a shared lumen (481) terminating at a shared port (481B) proximate the probe distal end; and
the instrument system includes:
a suction source (110);
an irrigation fluid source (112); and
a fluid connector manifold (478) fluidly connecting each of the suction source and the irrigation fluid source to the shared lumen;
the suction source is operable to generate a negative pressure in the shared lumen to execute an aspiration operation wherein the instrument system draws material into the shared lumen through the shared port; and
the irrigation fluid source fluidly is operable to execute a fluid delivery operation wherein the instrument system flows a fluid through the shared lumen and out through the shared port.

11. The instrument system of Claim 10 wherein:
the instrument includes an integral aspiration controller (464) operable to selectively control fluid flow between a suction source and the shared lumen, wherein the integral aspiration controller is mounted on the base; and
the instrument system includes a delivery controller (168) operable to selectively control fluid flow between the irrigation fluid source and the shared lumen.

12. The instrument system of Claim 11 wherein:
the instrument system includes a dilation controller (176) operable to selectively inflate and deflate the dilation balloon (474); and
the aspiration controller and the delivery controller are operable by the operator to execute the aspiration operation or the fluid delivery operation simultaneously while the dilation balloon is in its expanded configuration.

13. The instrument system of Claim 1 wherein:
the at least one lumen and the at least one port include:
an aspiration lumen (142) terminating at an inlet port (142B) proximate the probe distal end; and
a delivery lumen (144) terminating at an outlet port (144B) proximate the probe distal end; and
the instrument system includes:
a suction source (110) fluidly connected to the aspiration lumen and operable to generate a negative pressure in the aspiration lumen to execute an aspiration operation wherein the instrument system draws material into the aspiration lumen through the inlet port; and
an irrigation fluid source (112) fluidly connected to the delivery lumen and operable to execute a fluid delivery operation wherein the instrument system flows a fluid through the delivery lumen and out through the outlet port.

14. The instrument system of Claim 1 wherein the instrument includes an elastomeric atraumatic tip (488) mounted on the probe distal end.

15. The instrument system of Claim 1 wherein the instrument includes a visible dilation balloon position marker (479) located on the probe at a position along a length of the dilation balloon.

## Patentansprüche

1. Ein Instrumentensystem (400) zur Behandlung eines Individuums, wobei das Instrumentensystem Folgendes beinhaltet:
ein Instrument (420), das Folgendes einschließt:
eine Basis (422), die dazu ausgelegt ist, von einem Bediener ergriffen zu werden;
eine längliche Sonde (430), die ein proximales Sondenende (480A) einschließt, das mit der Basis gekoppelt ist und sich zu einem distalen Sondenende (480B) erstreckt, wobei die Sonde mindestens ein Lumen (481) einschließt, das an mindestens einer Anschlussöffnung (481B) endet, die in der Nähe des distalen Sondenendes liegt; und
einen Dilatationsballon (474), der an der Sonde in der Nähe des distalen Sondenendes angebracht ist, wobei der Dilatationsballon zu einer expandierten Konfiguration expandierbar ist, um einen Dilatationsvorgang auszuführen; und
ein integriertes Beleuchtungssystem (450), das Folgendes einschließt:
eine Lichtquelle (451); und
einen Wellenleiter (454) an der Sonde und der ein Licht aussendendes Ende (454B) in der Nähe des distalen Sondenendes aufweist, wobei das integrierte Beleuchtungssystem dazu ausgelegt ist, Licht von der Lichtquelle durch den Wellenleiter bis zu dem Licht aussendenden Ende durchzulassen;
wobei:
ein Abschnitt (454E) des Wellenleiters sich durch den Dilatationsballon erstreckt; und wobei das integrierte Beleuchtungssystem so ausgelegt ist, dass,
wenn die Lichtquelle betrieben wird, Licht (ER) von der Lichtquelle durch den Wellenleiter durchgelassen wird, radial nach außen durch eine Seitenwand des Wellenleiters ausgestrahlt wird und durch eine Seitenwand des Dilatationsballons durchgelassen wird.

2. Instrumentensystem gemäß Anspruch 1, wobei das integrierte Beleuchtungssystem eine Batterie (452) einschließt, um die Lichtquelle mit Strom zu versorgen, und optional
wobei die Lichtquelle und die Batterie in der Basis angebracht sind.

3. Instrumentensystem gemäß Anspruch 1, wobei die Lichtquelle (451) eine Leuchtdiode (LED) einschließt.

4. Instrumentensystem gemäß Anspruch 1, das ferner ein Endoskop (20) einschließt.

5. Instrumentensystem gemäß Anspruch 1, wobei das Instrument ein Wärmesenke-Element (453), das sich neben der Lichtquelle befindet, einschließt.

6. Instrumentensystem gemäß Anspruch 5, wobei:
das Wärmesenke-Element (453) eine Durchgangsbohrung (453A) einschließt; und
der Wellenleiter sich von der Lichtquelle durch die Durchgangsbohrung zu dem Licht aussendenden Ende erstreckt.

7. Instrumentensystem gemäß Anspruch 5, wobei das Wärmesenke-Element (453) aus Metall gebildet ist.

8. Instrumentensystem gemäß Anspruch 1, wobei:
der Abschnitt (454E) des Wellenleiters dazu ausgelegt ist, ein sich axial erstreckendes Lichtband durch die Seitenwand des Wellenleiters auszusenden; und
das Lichtband im Wesentlichen eine volle axiale Länge des Dilatationsballons (474) umspannt.

9. Instrumentensystem gemäß Anspruch 1, wobei:
die Sonde Folgendes einschließt:
einen länglichen, röhrenförmigen Außenschaft (432), wobei der Außenschaft starr ist; und
einen länglichen, röhrenförmigen Innenschaft (480), der sich durch den Außenschaft erstreckt,
wobei der Innenschaft verformbar ist;
der Innenschaft einen distalen Erweiterungsabschnitt (480E) einschließt, der sich distal über den Außenschaft hinaus zu dem distalen Sondenende hin erstreckt;
das mindestens eine Lumen sich durch den Innenschaft erstreckt; und
der Dilatationsballon (474) an dem distalen Erweiterungsabschnitt angebracht ist.

10. Instrumentensystem gemäß Anspruch 1, wobei:
das mindestens eine Lumen ein gemeinsames Lumen (481) einschließt, das an einer gemeinsamen Anschlussöffnung (481B) in der Nähe des distalen Sondenendes endet; und
das Instrumentensystem Folgendes einschließt:
eine Saugquelle (110);
eine Spülfluidquelle (112); und
einen Fluidverbinder-Verteiler (478), der jede von der Saugquelle und der Spülfluidquelle fluidisch mit dem gemeinsamen Lumen verbindet;
die Saugquelle betriebsfähig ist, um einen Unterdruck in dem gemeinsamen Lumen zu erzeugen, um einen Aspirationsvorgang auszuführen, wobei das Instrumentensystem Material durch die gemeinsame Anschlussöffnung in das gemeinsame Lumen hineinzieht; und
die Spülfluidquelle fluidisch betriebsfähig ist, um einen Fluidzuführungsvorgang auszuführen, wobei das Instrumentensystem ein Fluid durch das gemeinsame Lumen und durch die gemeinsame Anschlussöffnung herausströmen lässt.

11. Instrumentensystem gemäß Anspruch 10, wobei:
das Instrument eine integrierte Aspirationssteuereinheit (464) einschließt, die betriebsfähig ist, um den Fluidstrom zwischen einer Saugquelle und dem gemeinsamen Lumen selektiv zu steuern, wobei die integrierte Aspirationssteuereinheit an der Basis angebracht ist; und
das Instrumentensystem eine Zuführungssteuereinheit (168) einschließt, die betriebsfähig ist, um den Fluidstrom zwischen der Spülfluidquelle und dem gemeinsamen Lumen selektiv zu steuern.

12. Instrumentensystem gemäß Anspruch 11, wobei:
das Instrumentensystem eine Dilatationssteuereinheit (176) einschließt, die betriebsfähig ist, um den Dilatationsballon (474) selektiv aufzublasen und luftleer zu machen; und
die Aspirationssteuereinheit und die Zuführungssteuereinheit durch den Bediener betriebsfähig sind, um den Aspirationsvorgang oder den Fluidzuführungsvorgang gleichzeitig auszuführen, während der Dilatationsballon in seiner expandierten Konfiguration ist.

13. Instrumentensystem gemäß Anspruch 1, wobei:
das mindestens eine Lumen und die mindestens eine Anschlussöffnung Folgendes einschließen:
ein Aspirationslumen (142), das an einer Einlass-Anschlussöffnung (142B) in der Nähe des distalen Sondenendes endet; und
ein Zuführungslumen (144), das an einer Auslass-Anschlussöffnung (144B) in der Nähe des distalen Sondenendes endet; und
das Instrumentensystem Folgendes einschließt:
eine Saugquelle (110), die fluidisch mit dem Aspirationslumen verbunden ist und betriebsfähig ist, um einen Unterdruck in dem Aspirationslumen zu erzeugen, um einen Aspirationsvorgang auszuführen, wobei das Instrumentensystem Material durch die Einlass-Anschlussöffnung in das Aspirationslumen hineinzieht; und
eine Spülfluidquelle (112), die fluidisch mit dem Zuführungslumen verbunden ist und betriebsfähig ist, um einen Fluidzuführungsvorgang auszuführen, wobei das Instrumentensystem ein Fluid durch das Zuführungslumen und durch die Auslass-Anschlussöffnung herausströmen lässt.

14. Instrumentensystem gemäß Anspruch 1, wobei das Instrument eine elastomere atraumatische Spitze (488) einschließt, die an dem distalen Sondenende angebracht ist.

15. Instrumentensystem gemäß Anspruch 1, wobei das Instrument einen sichtbaren Dilatationsballon-Positionsmarker (479) einschließt, der sich auf der Sonde an einer Position entlang einer Länge des Dilatationsballons befindet.

## Revendications

1. Système d'instrument (400) destiné à traiter un sujet, le système d'instrument comprenant :
un instrument (420) comportant :
une base (422) configurée pour être saisie par un opérateur ;
une sonde (430) allongée comportant une extrémité proximale de sonde (480A) accouplée avec la base et s'étendant jusqu'à une extrémité distale de sonde (480B), la sonde comportant au moins une lumière (481) débouchant sur au moins un orifice (481B) à proximité de l'extrémité distale de sonde ; et
un ballonnet de dilatation (474) monté sur la sonde à proximité de l'extrémité distale de sonde, dans lequel le ballonnet de dilatation est expansible jusque dans une configuration expansée pour exécuter une opération de dilatation ; et
un système d'éclairage (450) intégré comportant :
une source de lumière (451) ; et
un guide d'ondes (454) sur la sonde et ayant une extrémité émettrice de lumière (454B) à proximité de l'extrémité distale de sonde, dans lequel le système d'éclairage intégré est configuré pour transmettre de la lumière à travers le guide d'ondes de la source de
lumière à l'extrémité émettrice de lumière ;
dans lequel :
une section (454E) du guide d'ondes s'étend à travers le ballonnet de dilatation ; et dans lequel le système d'éclairage intégré est configuré de sorte que
lorsque la source de lumière est mise en fonctionnement, de la lumière (ER) issue de la source de lumière est transmise à travers le guide d'ondes, émise radialement vers l'extérieur à travers une paroi latérale du guide d'ondes, et transmise à travers une paroi latérale du ballonnet de dilatation.

2. Système d'instrument selon la revendication 1 dans lequel le système d'éclairage intégré comporte une batterie (452) pour alimenter la source de lumière, et éventuellement,
dans lequel la source de lumière et la batterie sont montées dans la base.

3. Système d'instrument selon la revendication 1 dans lequel la source de lumière (451) comporte une diode électroluminescente (DEL).

4. Système d'instrument selon la revendication 1 comportant en outre un endoscope (20).

5. Système d'instrument selon la revendication 1 dans lequel l'instrument comporte un organe dissipateur thermique (453) situé adjacent à la source de lumière.

6. Système d'instrument selon la revendication 5 dans lequel :
l'organe dissipateur thermique (453) comporte un trou traversant (453A) ; et
le guide d'ondes s'étend à partir de la source de lumière à travers le trou traversant jusqu'à l'extrémité émettrice de lumière.

7. Système d'instrument selon la revendication 5 dans lequel l'organe dissipateur thermique (453) est formé de métal.

8. Système d'instrument selon la revendication 1 dans lequel :
la section (454E) du guide d'ondes est configurée pour émettre une bande en extension axiale de la lumière à travers la paroi latérale du guide d'ondes ; et
la bande de lumière couvre sensiblement une pleine longueur axiale du ballonnet de dilatation (474).

9. Système d'instrument selon la revendication 1 dans lequel :
la sonde comporte :
un arbre externe (432) tubulaire allongé, dans lequel l'arbre externe est rigide ; et
un arbre interne (480) tubulaire allongé s'étendant à travers l'arbre externe,
dans lequel l'arbre interne est malléable ;
l'arbre interne comporte une section d'extension distale (480E) s'étendant distalement au-delà de l'arbre externe vers l'extrémité distale de sonde ;
l'au moins une lumière s'étend à travers l'arbre interne ; et
le ballonnet de dilatation (474) est monté sur la section d'extension distale.

10. Système d'instrument selon la revendication 1 dans lequel :
l'au moins une lumière comporte une lumière partagée (481) débouchant sur un orifice partagé (481B) à proximité de l'extrémité distale de sonde ; et
le système d'instrument comporte :
une source de succion (110) ;
une source de fluide d'irrigation (112) ; et
un collecteur raccord de fluide (478) raccordant fluidiquement chacune de la source de succion et de la source de fluide d'irrigation à la lumière partagée ;
la source de succion peut être mise en fonctionnement pour générer une pression négative dans la lumière partagée pour exécuter une opération d'aspiration dans lequel le système d'instrument tire de la matière jusque dans la lumière partagée à travers l'orifice partagé ; et
la source de fluide d'irrigation peut être mise en fonctionnement fluidiquement pour exécuter une opération d'apport de fluide dans lequel le système d'instrument fait couler un fluide à travers la lumière partagée et vers l'extérieur à travers l'orifice partagé.

11. Système d'instrument selon la revendication 10 dans lequel :
l'instrument comporte un dispositif de commande d'aspiration (464) intégré pouvant être mis en fonctionnement pour commander sélectivement l'écoulement de fluide entre une source de succion et la lumière partagée, dans lequel le dispositif de commande d'aspiration intégré est monté sur la base ; et
le système d'instrument comporte un dispositif de commande d'apport (168) pouvant être mis en fonctionnement pour commander sélectivement l'écoulement de fluide entre la source de fluide d'irrigation et la lumière partagée.

12. Système d'instrument selon la revendication 11 dans lequel :
le système d'instrument comporte un dispositif de commande de dilatation (176) pouvant être mis en fonctionnement pour gonfler et dégonfler sélectivement le ballonnet de dilatation (474) ; et
le dispositif de commande d'aspiration et le dispositif de commande d'apport peuvent être mis en fonctionnement par l'opérateur pour exécuter l'opération d'aspiration ou l'opération d'apport de fluide simultanément tandis que le ballonnet de dilatation est dans sa configuration expansée.

13. Système d'instrument selon la revendication 1 dans lequel :
l'au moins une lumière et l'au moins un orifice comportent :
une lumière d'aspiration (142) débouchant sur un orifice d'entrée (142B) à proximité de l'extrémité distale de sonde ; et
une lumière d'apport (144) débouchant sur un orifice de sortie (144B) à proximité de l'extrémité distale de sonde ; et
le système d'instrument comporte :
une source de succion (110) raccordée fluidiquement à la lumière d'aspiration et pouvant être mise en fonctionnement pour générer une pression négative dans la lumière d'aspiration pour exécuter une opération d'aspiration dans lequel le système d'instrument tire de la matière jusque dans la lumière d'aspiration à travers l'orifice d'entrée ; et
une source de fluide d'irrigation (112) raccordée fluidiquement à la lumière d'apport et pouvant être mise en fonctionnement pour exécuter une opération d'apport de fluide dans lequel le système d'instrument fait couler un fluide à travers la lumière d'apport et vers l'extérieur à travers l'orifice de sortie.

14. Système d'instrument selon la revendication 1 dans lequel l'instrument comporte une pointe atraumatique (488) élastomère montée sur l'extrémité distale de sonde.

15. Système d'instrument selon la revendication 1 dans lequel l'instrument comporte un marqueur (479) de position de ballonnet de dilatation visible situé sur la sonde à une position suivant une longueur du ballonnet de dilatation.
